(19) Europäisches Patentamt
European Patent Office
Office européen des brevets

(11) EP 2 830 709 B1

(12) **EUROPÄISCHE PATENTSCHRIFT**

(45) Veröffentlichungstag und Bekanntmachung des Hinweises auf die Patenterteilung:
06.05.2020 Patentblatt 2020/19

(21) Anmeldenummer: 13729667.9

(22) Anmeldetag: 11.06.2013

(51) Int Cl.:
*A61N 5/10* (2006.01)

(86) Internationale Anmeldenummer:
PCT/EP2013/061956

(87) Internationale Veröffentlichungsnummer:
WO 2014/009076 (16.01.2014 Gazette 2014/03)

(54) **VERFAHREN UND VORRICHTUNG ZUM BESTIMMEN EINES BESTRAHLUNGSPLANS FÜR EINE PARTIKELBESTRAHLUNGSANLAGE**

METHOD AND DEVICE FOR DETERMINING AN IRRADIATION PLAN FOR A PARTICLE IRRADIATION UNIT

PROCÉDÉ ET DISPOSITIF DE DÉTERMINATION D'UN PLAN DE RAYONNEMENT POUR UNE INSTALLATION DE RAYONNEMENT PARTICULAIRE

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 13.07.2012 DE 102012212340

(43) Veröffentlichungstag der Anmeldung:
04.02.2015 Patentblatt 2015/06

(73) Patentinhaber: Siemens Healthcare GmbH
91052 Erlangen (DE)

(72) Erfinder:
• BOHSUNG, Jörg
69126 Heidelberg (DE)
• ELSÄSSER, Thilo
91054 Buckenhof (DE)
• GRÖZINGER, Sven Oliver
91353 Hausen (DE)
• KIM, Johann
91056 Erlangen (DE)
• NEUHAUSER, Robert
85375 Neufahrn (DE)
• RIETZEL, Eike
64331 Weiterstadt (DE)
• SCHWEIZER, Bernd
68775 Ketsch (DE)
• THILMANN, Oliver
86159 Augsburg (DE)

(56) Entgegenhaltungen:
EP-A1- 2 510 978          DE-A1-102008 009 765
DE-B3-102011 088 160

**Beschreibung**

Die vorliegende Erfindung betrifft ein Verfahren und eine Vorrichtung zur Bestimmung eines Bestrahlungsplans für eine Partikelbestrahlungsanlage und eine entsprechend ausgestaltete Partikelbestrahlungsanlage.

Beispielsweise im Rahmen einer Therapieplanung bei der Partikeltherapie wird im Vorfeld ein Bestrahlungsplan erstellt, welcher Steuerparameter zur Bestrahlung eines Untersuchungsobjekts definiert. Mit dem Bestrahlungsplan wird die Bestrahlung eines Gegenstands gemäß bestimmten Vorgaben (z. B. Zielvolumen oder Dosisverteilung) geplant.

Die Partikeltherapie ist ein mittlerweile etabliertes Verfahren, mit welchem insbesondere von Tumorerkrankungen befallenes Gewebe bestrahlt wird. Bei der Partikeltherapie werden geladene Partikel, wie beispielsweise Protonen oder Kohlenstoff-Ionen oder andere Ionen auf hohe Energien beschleunigt, zu einem Partikelstrahl geformt und über ein Hochenergie-Transportsystem zu einem (oder mehreren) Bestrahlungsräumen geführt. In einem Bestrahlungsraum wird das Zielvolumen des Behandlungsobjekts mit dem Partikelstrahl bestrahlt, wobei zwangsweise auch Gewebe außerhalb des Zielvolumens bestrahlt wird.

Wenn bei der Partikeltherapie Beschleuniger mit einer aktiven Energievariation eingesetzt werden, werden für die Bestrahlung des Zielvolumens Teilchenstrahlen unterschiedlicher Energie eingesetzt, wodurch sich so genannte Isoenergieschichten ausbilden, welche innerhalb und außerhalb des Zielvolumens liegen können. In einer Isoenergieschicht werden von dem Teilchenstrahl Teilchen derselben Energie appliziert, so dass die Teilchen des Teilchenstrahls zur Einstellung auf die jeweilige Isoenergieschicht jeweils eine Energie aufweisen, welche sich von der Energie der Teilchen für andere Isoenergieschichten unterscheidet. Dabei werden nach Möglichkeit alle Teilchen einer Isoenergieschicht mit Hilfe eines Spills (d.h. mittels einer einzigen Beschleunigerfüllung) appliziert, da das Laden bzw. Erzeugen eines neuen Spills mehrere Sekunden dauert. Die Unterbrechungszeit zur Erzeugung eines neuen Spills macht insgesamt abhängig von der zu applizierenden Teilchenzahl ungefähr die Hälfte der Gesamtbestrahlungsdauer aus. Um eine möglichst kurze patientenfreundliche Bestrahlung, eine genaue Applikation der Dosis im Zielvolumen sowie einen möglichst wirtschaftlichen Betrieb der Partikelbestrahlungsanlage zu gewährleisten, muss die direkte Bestrahlungszeit sowie die Gesamtbestrahlungsdauer so kurz wie möglich gehalten werden. Dabei muss jedoch die Qualität des Bestrahlungsplans beachtet werden, welche letztlich vom behandelnden Arzt zu beurteilen ist.

Daher stellt sich die vorliegende Erfindung die Aufgabe, die Gesamtbestrahlungsdauer unter Beibehaltung einer akzeptablen Planqualität bei der Bestimmung eines Bestrahlungsplans für eine Partikelbestrahlungsanlage möglichst kurz zu halten.

Erfindungsgemäß wird diese Aufgabe durch ein Verfahren zum Bestimmen eines Bestrahlungsplans für eine Partikelbestrahlungsanlage nach Anspruch 1, durch eine Vorrichtung zum Bestimmen eines Bestrahlungsplans für eine Partikelbestrahlungsanlage nach Anspruch 7, durch eine Partikelbestrahlungsanlage nach Anspruch 9, durch ein Computerprogrammprodukt nach Anspruch 10 oder durch einen elektronisch lesbaren Datenträger nach Anspruch 11 gelöst. Die abhängigen Ansprüche definieren bevorzugte und vorteilhafte Ausführungsformen der vorliegenden Erfindung.

Im Rahmen der vorliegenden Erfindung wird ein Verfahren zum (insbesondere automatischen) Bestimmen eines Bestrahlungsplans für eine Partikelbestrahlungsanlage bereitgestellt. Dabei wird mit Hilfe der Partikelbestrahlungsanlage gemäß dem Bestrahlungsplan ein Zielvolumen innerhalb eines Untersuchungsobjekts mit einem Partikelstrahl bzw. Teilchenstrahl bestrahlt. Der Bestrahlungsplan wird dabei in einem ersten Durchlauf ausgehend von einem vorgegebenen Zielvolumen und einer vorbestimmten Dosisverteilung (Soll-Dosisverteilung) innerhalb dieses Zielvolumens bestimmt, um anhand des Bestrahlungsplans die Dosis des Teilchenstrahls mit einer hohen Qualität (z. B. möglichst exakt entsprechend der Soll-Dosisverteilung) in dem Zielvolumen, welches mehrere Isoenergieschichten umfasst, zu deponieren oder zu applizieren. Darüber hinaus wird der Bestrahlungsplan in einem zweiten Durchlauf derart bestimmt, dass eine oder mehrere der Isoenergieschichten nicht berücksichtigt und damit nicht bestrahlt werden. Die durch die Nicht-Berücksichtigung von mindestens einer Isoenergieschicht entstehenden Dosislücken können dann beispielsweise durch die Änderung der Dosis für andere Isoenergieschichten kompensiert werden. Dabei wird die eine oder werden die mehreren Isoenergieschichten (im Folgenden n Isoenergieschichten genannt) nach einem oder nach mehreren der folgenden Kriterien bestimmt:

    1. Kriterium:

        Es werden diejenigen n Isoenergieschichten mit der niedrigsten Energie nicht berücksichtigt und damit nicht bestrahlt. Anders ausgedrückt werden diejenigen n Isoenergieschichten nicht bestrahlt, zu deren Bestrahlung der Teilchenstrahl eine Energie aufweisen würde, welche unterhalb einer minimalen Grenzenergie liegt.

        Die minimale Grenzenergie kann dabei absolut (d.h. ohne Berücksichtigung der jeweiligen Energie der Isoenergieschichten) oder relativ (d.h. unter Berücksichtigung der jeweiligen Energie der Isoenergieschichten) definiert werden. Wenn beispielsweise die drei (n) Isoenergieschichten mit der niedrigsten Energie nicht berücksichtigt bzw. bestrahlt werden sollen, wird die minimale Grenzenergie relativ zu der Energie der Isoenergieschicht mit der drittniedrigsten (n-niedrigsten) Energie definiert und liegt z.B. leicht oberhalb der Energie (der Teilchen) der Isoenergieschicht mit der drittniedrigsten (n-niedrigsten) Energie. Dagegen berücksichtigt eine absolute Definition der minimalen Grenzenergie die jeweilige Energie der Isoenergieschichten nicht, so dass beispielsweise alle Isoenergieschichten unterhalb einer vorbestimmten Energie (z.B. 150 MeV) nicht bestrahlt werden, wobei dieses Kriterium unabhängig davon ist, wie viele oder welcher Prozentsatz der Isoenergieschichten dann nicht bestrahlt werden.

2. Kriterium:

Es werden diejenigen n Isoenergieschichten mit der größten Energie nicht berücksichtigt und damit nicht bestrahlt. Anders ausgedrückt werden diejenigen n Isoenergieschichten nicht bestrahlt, zu deren Bestrahlung der Teilchenstrahl eine Energie aufweisen würde, welcher oberhalb einer maximalen Grenzenergie liegt.

Die maximale Grenzenergie kann dabei absolut (d.h. ohne Berücksichtigung der jeweiligen Energie der Isoenergieschichten) oder relativ (d.h. unter Berücksichtigung der jeweiligen Energie der Isoenergieschichten) definiert werden. Wenn beispielsweise die drei (n) Isoenergieschichten mit der höchsten Energie nicht berücksichtigt bzw. bestrahlt werden sollen, wird die maximale Grenzenergie relativ zu der Energie der Isoenergieschicht mit der dritthöchsten (n-höchsten) Energie definiert und liegt leicht unterhalb der Energie (der Teilchen) der Isoenergieschicht mit der dritthöchsten (n-höchsten) Energie. Dagegen berücksichtigt eine absolute Definition der maximalen Grenzenergie die jeweilige Energie der Isoenergieschichten nicht.

3. Kriterium:

Es werden diejenigen n Isoenergieschichten nicht bestrahlt, welche gemäß dem Bestrahlungsplan nach dem ersten Durchlauf die kleinste Anzahl von Rasterpunkten der Isoenergieschichten aufweisen. Mit anderen Worten wird die Rasterpunktanzahl nach dem ersten Durchlauf für jede Isoenergieschicht bestimmt, und es werden diejenigen n Isoenergieschichten ermittelt, bei denen diese Rasterpunktanzahl im Vergleich zu den anderen Isoenergieschichten am geringsten ist. Diese derart ermittelten n Isoenergieschichten werden nicht berücksichtigt bzw. bestrahlt.

Unter einem Rasterpunkt wird dabei ein Punkt in einer zum Teilchenstrahl orthogonalen Ebene durch das Isozentrum verstanden. Der Teilchenstrahl wird jeweils auf einen Rasterpunkt gerichtet, welcher somit die Richtung des Teilchenstrahls bestimmt.

4. Kriterium:
Es werden diejenigen n Isoenergieschichten nicht bestrahlt, welche gemäß dem Bestrahlungsplan nach dem ersten Durchlauf die kleinste Gesamtpartikelanzahl der Isoenergieschichten aufweisen. Anders ausgedrückt wird die Gesamtpartikelanzahl nach dem ersten Durchlauf für jede Isoenergieschicht ermittelt, und es werden diejenigen n Isoenergieschichten bestimmt, bei denen diese Gesamtpartikelanzahl im Vergleich zu den anderen Isoenergieschichten am geringsten ist. Diese derart bestimmten n Isoenergieschichten werden nicht berücksichtigt bzw. bestrahlt.

5. Kriterium:
Es werden diejenigen n Isoenergieschichten nicht bestrahlt, welche gemäß dem Bestrahlungsplan nach dem ersten Durchlauf den geringsten Dosisbeitrag zu der im Zielvolumen zu applizierenden Gesamtdosis aufweisen. Anders ausgedrückt wird für jede Isoenergieschicht nach dem ersten Durchlauf die von der jeweiligen Isoenergieschicht im Zielvolumen zu applizierende Dosis bestimmt, und es werden diejenigen n Isoenergieschichten bestimmt, bei denen diese Dosis im Vergleich zu den anderen Isoenergieschichten am kleinsten ist. Diese derart bestimmten n Isoenergieschichten werden nicht berücksichtigt bzw. bestrahlt.

6. Kriterium:
Es werden diejenigen n Isoenergieschichten nicht bestrahlt, welche gemäß dem Bestrahlungsplan nach dem ersten Durchlauf den geringsten Beitrag zu einer Zielfunktion liefern, welche zur Bestimmung des Bestrahlungsplans optimiert wird. Anders ausgedrückt wird für jede Isoenergieschicht nach dem ersten Durchlauf der Beitrag zu der Zielfunktion bestimmt, und es werden diejenigen n Isoenergieschichten bestimmt, bei denen dieser Beitrag im Vergleich zu den anderen Isoenergieschichten am geringsten ist. Diese derart bestimmten n Isoenergieschichten werden nicht berücksichtigt bzw. bestrahlt.

7. Kriterium:

Es werden diejenigen n Isoenergieschichten nicht bestrahlt, welche gemäß dem Bestrahlungsplan nach dem ersten Durchlauf den kleinsten Dosiskompensationsfehler aufweisen. Anders ausgedrückt wird für jede Isoenergieschicht nach dem ersten Durchlauf der Dosiskompensationsfehler bestimmt, und es werden diejenigen n Isoenergieschichten bestimmt, bei denen dieser Dosiskompensationsfehler im Vergleich zu den anderen Isoenergieschichten am kleinsten ist. Diese derart bestimmten n Isoenergieschichten werden nicht berücksichtigt bzw. bestrahlt.

8. Kriterium:

Es werden diejenigen n Isoenergieschichten nicht bestrahlt, deren Weglassen nach einer erneuten Optimierung zu der kleinsten Änderung des Zielfunktionswerts führt. Anders ausgedrückt werden diejenigen n Isoenergieschichten nicht bestrahlt, bei denen der mit ihrem Weglassen verbundene Dosisverlust am besten durch andere Isoenergieschichten kompensiert werden kann. Zur Ermittlung dieser n Isoenergieschichten wird insbesondere eine vollständige Optimierung der Zielfunktion unter der Voraussetzung, dass jeweils n Isoenergieschichten nicht bestrahlt werden, durchgeführt.

Mit anderen Worten wird für verschiedene Mengen von jeweils n Isoenergieschichten jeweils der Zielfunktionswert unter der Voraussetzung mittels einer Optimierung erstellt, dass die jeweilige Menge von n Isoenergieschichten nicht bestrahlt wird. Aus diesen Mengen wird schließlich diejenige Menge von n Isoenergieschichten gewählt, bei der die Änderung des Zielfunktionswerts im Vergleich zu einem Zielfunktionswert am geringsten ist, welcher unter der Voraussetzung mittels einer Optimierung erstellt wird, dass auch die jeweilige Menge von n Isoenergieschichten bestrahlt wird.

Die Zahl n ist dabei für jedes Kriterium eine natürliche Zahl, was bedeutet, dass auch nur eine Isoenergieschicht nicht berücksichtigt bzw. bestrahlt werden kann.

Darüber hinaus kann die Bestimmung der nicht zu bestrahlenden Isoenergieschichten bei den Kriterien 1 und 2 auch nur in einem Durchlauf (anstelle des ersten und des zweiten Durchlaufs) erfolgen, da bereits vor der Bestimmung des Bestrahlungsplans die Energie des Teilchenstrahls für die jeweilige Isoenergieschicht bekannt ist, so dass der Bestrahlungsplan quasi bereits im ersten Durchlauf unter der Voraussetzung erstellt werden kann, dass die n Isoenergieschichten gemäß dem Kriterium 1 und/oder 2 nicht bestrahlt werden. Generell gilt darüber hinaus, dass die Anzahl der potentiell zu bestrahlenden Isoenergieschichten schon im ersten Durchlauf oder vor dem ersten Durchlauf bereits beispielsweise abhängig von einer zu bestrahlenden Tumorgröße reduziert werden kann.

Zur Bestimmung des Bestrahlungsplans wird die Zielfunktion optimiert, wobei jede Isoenergieschicht einen bestimmten Beitrag zu dieser Zielfunktion liefert. Der Beitrag der jeweiligen Isoenergieschicht kann sich dabei aus einer Summe aus positiven Beiträgen (z. B. Dosisbeitrag zur Gesamtdosis) und aus negativen Beiträgen (Zeitdauer der Bestrahlung, Bestrahlung gesunden Gewebes) zusammensetzen. Bei der Ermittlung der n Isoenergieschichten mit dem geringsten Beitrag zur Zielfunktion kann somit das Vorzeichen des jeweiligen Beitrags der jeweiligen Isoenergieschicht berücksichtigt werden.

Die Zielfunktion kann dabei einen Bestrafungsterm bzw. negativen Beitrag umfassen, wobei dieser Bestrafungsterm umso größer ist, je mehr Isoenergieschichten bestrahlt werden.

Wenn eine Isoenergieschicht oder mehrere Isoenergieschichten nicht bestrahlt werden, muss der durch den Wegfall dieser Isoenergieschichten entfallende Dosisbeitrag durch eine entsprechende Dosiserhöhung der zu bestrahlenden Isoenergieschichten aufgefangen werden. Zur Bestimmung des Dosiskompensationsfehlers kann eine erste Dosisverteilung unter der Annahme bestimmt werden, dass sowohl die zu bestrahlenden als auch die nicht zu bestrahlenden Isoenergieschichten bestrahlt werden. Eine zweite Dosisverteilung wird unter der Annahme bestimmt, dass nur die zu bestrahlenden Isoenergieschichten bestrahlt werden, wobei diese Isoenergieschichten zum Ausgleich der nicht bestrahlten Isoenergieschichten eine entsprechend höhere Dosis aufweisen. Der Dosiskompensationsfehler entspricht dann beispielsweise einer Differenz zwischen der ersten Dosisverteilung und der zweiten Dosisverteilung.

Die folgende Gleichung (1) bestimmt die an einem beliebigen Punkt i ("Point of Interest") im Zielvolumen vorliegende bzw. auftretende Dosisdifferenz $r_i(IES)$.

$$r_i(IES) = d_i - d_i(IES) \qquad (1)$$

Diese Dosisdifferenz $r_i(IES)$ berechnet sich aus der Differenz der für den Punkt i geplanten Soll-Dosis $d_i$ abzüglich des Soll-Dosis-Anteils $d_i(IES)$, welcher durch die Bestrahlung der bestimmten Isoenergieschicht IES hervorgerufen wird.

Ausgehend von der Gleichung (1) kann ein globaler Kompensationsfaktor c derart bestimmt werden, dass ein Produkt aus der Dosisdifferenz $r_i$(IES) und diesem Kompensationsfaktor möglichst an jedem Punkt i des Zielvolumens der für den entsprechenden Punkt geltenden Soll-Dosis $d_i$ entspricht.

Aber auch wenn der Wegfall einer bestimmten Isoenergieschicht IES kompensiert wird, indem die für jeden Rasterpunkt der noch zu bestrahlenden Isoenergieschichten bestimmte Teilchenzahl mit diesem Kompensationsfaktor c multipliziert wird, tritt ein minimaler Fehler err(IES) (im Folgenden auch Dosiskompensationsfehler genannt) auf, welcher gemäß der Gleichung (2) bestimmt werden kann.

$$err(IES) = \min_c \sum_i (c \times r_i(IES) - d_i)^2 \qquad (2)$$

Zur Bestimmung des minimalen Fehlers err(IES) für eine bestimmte Isoenergieschicht IES wird demnach der optimale Kompensationsfaktor $c_{opt}$ bestimmt, bei welchem der Dosiskompensationsfehler minimal ist. Dieser optimale Kompensationsfaktor $c_{opt}$ kann anhand der notwendigen Bedingung, dass die erste Ableitung nach c der Dosiskompensationsfehler-Funktion err(IES) nach Gleichung (2) den Wert 0 aufweisen muss, bestimmt werden. Dadurch kann der optimale Kompensationsfaktor $c_{opt}$ durch die folgende Gleichung (3) bestimmt werden.

$$c_{opt} = \frac{\sum_i d_i \times r_i(IES)}{\sum_i (r_i(IES))^2} \qquad (3)$$

Für diesen optimalen Kompensationsfaktor $c_{opt}$ ergibt sich dann der entsprechende minimale Dosiskompensationsfehler err(IES) für die entsprechende Isoenergieschicht IES nach Gleichung (4) oder (5).

$$err(IES) = \sum_i d_i^2 - \frac{\left(\sum_i d_i \times r_i(IES)\right)^2}{\sum_i (r_i(E))^2} \qquad (4)$$

$$err(IES) = \left(1 - \frac{\left(\sum_i d_i \times r_i(IES)\right)^2}{\sum_i d_i^2 \times \sum_i (r_i(E))^2}\right) \times \sum_i d_i^2 \qquad (5)$$

Zur Bestimmung derjenigen Isoenergieschicht, nach welcher der nach Gleichung (2), (4) oder (5) bestimmte Dosiskompensationsfehler am geringsten ist, kann beispielsweise für jede Isoenergieschicht der Dosiskompensationsfehler nach der Gleichung (2), (4) bzw. (5) bestimmt werden und dann diejenige Isoenergieschicht gewählt werden, bei welcher der Dosiskompensationsfehler err(IES) den geringsten Wert aufweist.

Die vorab beschriebenen Gleichungen (2) bis (5) setzen voraus, dass nur ein globaler Kompensationsfaktor c bzw. $c_{opt}$ existiert, welcher für alle zu bestrahlende Isoenergieschichten derselbe ist. Zur Kompensation der wegzulassenden Isoenergieschicht IES werden die pro Rasterpunkt geplanten Teilchenzahlen für jede zu bestrahlende Isoenergieschicht mit diesem Kompensationsfaktor multipliziert.

Gemäß einer erfindungsgemäßen Variante ist es allerdings auch möglich, für jede zu bestrahlende Isoenergieschicht einen individuellen Kompensationsfaktor zu bestimmen, wodurch es beispielsweise möglich ist, insbesondere diejenigen Isoenergieschichten mit einem größeren Kompensationsfaktor zu Beaufschlagung, welche in der Nähe der wegzulassenden Isoenergieschicht liegen.

Zur möglichst optimalen Wahl dieser individuellen Kompensationsfaktoren kann beispielsweise die quadratische Abweichung F von der Soll-Dosis $d_i$ für jeden relevanten Punkt i im Zielvolumen anhand der folgenden Gleichung (6) bestimmt werden.

$$F = \sum_i \left( k_i - d_i \right)^2 \qquad\qquad (6)$$

Bei dieser Gleichung (6) entspricht $k_i$ der Ist-Dosis im Punkt i.

Um nun zu bestimmen, welche Isoenergieschicht am besten weggelassen werden könnte, wird beispielsweise für jede Isoenergieschicht, welche laut dem aktuellen Bestrahlungsplan noch zu bestrahlen ist, derjenige Satz von individuellen Kompensationsfaktoren (für die zu bestrahlenden Isoenergieschichten) bestimmt, bei welchem die in Gleichung (6) angegebene quadratische Abweichung F den kleinsten Wert aufweist. Ausgehend davon wird nun diese Isoenergieschicht weggelassen, bei welcher die quadratische Abweichung F den minimalen kleinsten Wert unter allen Isoenergieschichten aufweist.

Erfindungsgemäß ist es dabei möglich, das Weglassen einer Isoenergieschicht derart zu simulieren, dass ihr Weglassen durch eines von den folgenden Vorgehen kompensiert wird:

- Mit einem globalen Kompensationswert wird die Dosis der zu bestrahlenden Isoenergieschichten erhöht, um das Weglassen der jeweiligen Isoenergieschicht zu kompensieren.
- Mittels einer schnellen Optimierung wird pro Isoenergieschicht ein individueller Kompensationsfaktor bestimmt, um den die Dosis der jeweiligen zu bestrahlenden Isoenergieschicht erhöht wird, um das Weglassen der jeweiligen Isoenergieschicht zu kompensieren.
- Anhand einer vollständigen Optimierung wird unter der Voraussetzung, dass die jeweilige Isoenergieschicht weggelassen wird, ein fiktiver Bestrahlungsplan erstellt, so dass die Ist-Dosis für jede zu bestrahlende Isoenergieschicht neu bestimmt wird.

Mit anderen Worten gibt es erfindungsgemäß mehrere Möglichkeiten, welche sich neben der Qualität im Rechenaufwand unterscheiden, um diejenige Isoenergieschicht zu bestimmen, deren Weglassen zu der kleinsten quadratischen Abweichung F unter allen Isoenergieschichten führt.

Es sei darauf hingewiesen, dass in einem Schritt nicht nur eine, sondern mehrere Isoenergieschichten von der Bestrahlung ausgenommen werden können. Dies gilt sowohl für die mit einem globalen Kompensationsfaktor arbeitende Variante als auch für die mit individuellen Kompensationsfaktoren arbeitende Variante wie auch für die oben skizzierten Vorgehen. Bei beiden Varianten und den oben skizzierten Vorgehen wird aus der Obermenge der laut dem aktuellen Bestrahlungsplan zu bestrahlenden Isoenergieschichten diejenige Teilmenge ausgewählt, deren Ausfall (bei der Bestrahlung) beispielsweise zu der geringsten quadratischen Abweichung F nach Gleichung (6) führen würde.

Zur Bestimmung der n nicht zu bestrahlenden Isoenergieschichten gemäß einem oder mehreren der vorab beschriebenen Kriterien kann auch ein Verhältnis der Anzahl bzw. des Anteils entsprechend des jeweiligen Kriteriums der n Isoenergieschichten zu einer Summe der Anzahlen bzw. der Anteile entsprechend des jeweiligen Kriteriums aller Isoenergieschichten gebildet werden. In diesem Fall werden die n Isoenergieschichten derart bestimmt, dass dieses Verhältnis gerade noch unterhalb eines vorbestimmten Schwellenwerts liegt, so dass ein Hinzufügen einer weiteren Isoenergieschicht zu den n Isoenergieschichten dazu führt, dass das Verhältnis oberhalb des vorbestimmten Schwellenwerts liegt.

Beispielsweise kann bei dem Kriterium 3 das Verhältnis derart gebildet werden, dass die Summe der Rasterpunktanzahlen der n Isoenergieschichten zu der Summe der Rasterpunktanzahlen aller Isoenergieschichten ins Verhältnis gesetzt wird. In diesem Fall setzen sich die n Isoenergieschichten aus denjenigen Isoenergieschichten mit den kleinsten Rasterpunktanzahlen zusammen, so dass das beschriebene Verhältnis gerade noch unterhalb des vorbestimmten Schwellenwerts (z.B. 30 % bzw. 0,3) liegt.

Bei dem Kriterium 5 kann das Verhältnis derart gebildet werden, dass die Summe der Dosisbeiträge der n Isoenergieschichten zu der Gesamtdosis (entspricht der Summe der Dosisbeiträge aller Isoenergieschichten) ins Verhältnis gesetzt wird. In diesem Fall setzen sich die n Isoenergieschichten aus denjenigen Isoenergieschichten mit den kleinsten Dosisbeiträgen zusammen, so dass das beschriebene Verhältnis gerade noch unterhalb des vorbestimmten Schwellenwerts (z.B. 0,2) liegt.

Die Bestimmung der n Isoenergieschichten kann auch mit Bezug zu einem Maximalwert erfolgen. Dazu wird die Summe der Anzahlen (Rasterpunktanzahlen oder Gesamtpartikelanzahlen) bzw. der Anteile der n Isoenergieschichten entsprechend des jeweiligen Kriteriums gebildet. Die n Isoenergieschichten werden dabei derart gewählt, dass die Summe gerade noch unterhalb eines vorbestimmten Maximalwerts liegt, so dass ein Hinzufügen einer weiteren der Isoenergieschichten zu der Menge der n Isoenergieschichten dazu führt, dass die Summe oberhalb des vorbestimmten Maximalwert liegt.

Beispielsweise kann bei dem Kriterium 4 eine maximale Summe von Gesamtpartikelanzahlen vorgegeben werden. Die n Isoenergieschichten mit der geringsten Gesamtpartikelanzahl werden in diesem Fall so bestimmt, dass die Summe der Gesamtpartikelanzahlen dieser n Isoenergieschichten gerade noch unter der vorgegebenen maximalen Summe liegt.

In ähnlicher Weise kann bei dem Kriterium 6 ein maximaler Betrag zur Zielfunktion vorgegeben werden. Die n Isoenergieschichten mit den geringsten Beiträgen zur Zielfunktion werden in diesem Fall so bestimmt, dass die Summe der Beiträge zur Zielfunktion dieser n Isoenergieschichten gerade noch unter dem maximalen Betrag liegt.

Gemäß einer bevorzugten erfindungsgemäßen Ausführungsform wird eine von dem Bestrahlungsplan einzuhaltende Bedingung vorgegeben. Die Menge der n Isoenergieschichten, welche nicht bestrahlt wird, wird nun solange gemäß einem der vorab beschriebenen Kriterien um weitere Isoenergieschichten erweitert, solange diese Bedingung noch von dem Bestrahlungsplan eingehalten wird. Beispielsweise kann schrittweise gemäß einem der vorab beschriebenen Kriterien eine weitere Isoenergieschicht der n Isoenergieschichten hinzugefügt werden, bis der Bestrahlungsplan, welcher ausgehend von den zu bestrahlenden Isoenergieschichten (also ohne die aktuelle Menge der n Isoenergieschichten) erstellt wird, die vorgegebene Bedingung nicht mehr erfüllt. Die im letzten Schritt hinzugefügte Isoenergieschicht wird dann von der Menge der n Isoenergieschichten entfernt, um den Bestrahlungsplan zu bestimmen.

Bei der vorgegebenen Bedingung kann es sich beispielsweise um die Einhaltung bestimmter Toleranzgrenzen von Risikoorganen, um durch Dosis-Volumen-Histogramme definierte Bedingungen, um die Einhaltung von Toleranzgrenzen hinsichtlich der Dosisverteilung innerhalb des Zielvolumens oder die Übereinstimmung zwischen tatsächlicher Dosisverteilung und Soll-Dosisverteilung sowie um eine Kombination vorab genannter Bedingungen handeln.

Erfindungsgemäß ist es auch möglich, dass der Bestrahlungsplan in mehr als zwei Durchläufen erstellt wird. Dazu werden nach jedem Durchlauf (vielleicht bis auf den letzten Durchlauf) nach dem jeweiligen Kriterium jeweils n Isoenergieschichten bestimmt, welche nicht bestrahlt werden, was dann im jeweils nächsten Durchlauf zur Bestimmung des Bestrahlungsplans berücksichtigt wird. Die Bestimmung der nach jedem Durchlauf hinzukommenden nicht zu bestrahlenden Isoenergieschichten werden dabei abhängig von Eigenschaften (z.B. Rasterpunktanzahl, Gesamtpartikelanzahl) der Isoenergieschichten bestimmt, welche durch den zuletzt bestimmten Bestrahlungsplan bestimmt worden sind.

Mit anderen Worten erfolgt die Auswahl der nächsten nicht zu bestrahlenden Isoenergieschicht(en) nur unter Berücksichtigung der noch übrig gebliebenen Isoenergieschichten bzw. den noch zur Bestrahlung vorgesehenen Isoenergieschichten. Es wird ausgehend von diesen Isoenergieschichten ein Bestrahlungsplan bestimmt und dann gemäß des oder der gewählten Kriterien die nächsten nicht zu bestrahlenden Isoenergieschichten bestimmt, welche dann der Menge der nicht zu bestrahlenden Isoenergieschichten hinzugefügt werden bzw. aus der Menge der zu bestrahlenden Isoenergieschichten entfernt werden.

Obiger Sachverhalt, den Bestrahlungsplan in mehr als zwei Durchläufen zu erstellen, wird im Folgenden nochmals beschrieben.

Wenn der Bestrahlungsplan in mehr als zwei Durchläufen erstellt wird, werden in jedem Durchlauf nach den jeweiligen Kriterien Isoenergieschichten bestimmt, welche im weiteren Verlauf der Bestrahlungsplan-Erstellung nicht mehr berücksichtigt und damit auch nicht bestrahlt werden. Jeder Durchlauf (bis auf den ersten Durchlauf) basiert demnach auf dem Planungsergebnis des vorangegangenen Durchlaufs, wobei zwischen zwei Durchläufen weitere Operationen zur Optimierung des Bestrahlungsplans vorgenommen werden können. Ein Beispiel einer solchen Operation ist eine Neuoptimierung des Bestrahlungsplans auf Basis der für den jeweiligen Durchlauf aktuellen (zu bestrahlenden) Isoenergieschichten.

Im Rahmen der vorliegenden Erfindung wird auch ein weiteres Verfahren zum Bestimmen eines Bestrahlungsplans für eine Partikelbestrahlungsanlage bereitgestellt. Auch dabei wird mit der Partikelbestrahlungsanlage abhängig von dem Bestrahlungsplan ein Zielvolumen innerhalb eines Untersuchungsobjekts mit einem Teilchenstrahl bestrahlt, wobei das Zielvolumen und eine vorbestimmte Dosisverteilung vorgegeben sind. Der Bestrahlungsplan wird in einem ersten Durchlauf bestimmt, um den Teilchenstrahl gemäß der vorbestimmten Dosisverteilung in dem Zielvolumen, welches mehrere Isoenergieschichten umfasst, zu applizieren. In einem zweiten Durchlauf wird der Bestrahlungsplan zusätzlich unter der Bedingung bestimmt, so dass nur ausgewählte bzw. bestimmte der Isoenergieschichten bestrahlt werden, welche nach mindestens einem von folgenden Kriterien bestimmt werden:

1. Kriterium:
Es werden nur diejenigen Isoenergieschichten bestimmt und damit bestrahlt, zu deren Bestrahlung der Teilchenstrahl eine oberhalb einer minimalen Grenzenergie liegende Energie aufweist.

2. Kriterium:
Es werden nur diejenigen Isoenergieschichten bestimmt und damit bestrahlt, zu deren Bestrahlung der Teilchenstrahl eine unterhalb einer maximalen Grenzenergie liegende Energie aufweist.

3. Kriterium:
Es werden nur diejenigen Isoenergieschichten bestimmt und damit bestrahlt, welche laut dem nach dem ersten Durchlauf erstellten Bestrahlungsplan die größte Anzahl von Rasterpunkten im Vergleich zu den anderen Isoenergieschichten aufweisen.

4. Kriterium:
Es werden nur diejenigen Isoenergieschichten bestimmt und damit bestrahlt, welche laut dem nach dem ersten Durchlauf erstellten Bestrahlungsplan die größte Gesamtpartikelanzahl im Vergleich zu den anderen Isoenergieschichten aufweisen.

5. Kriterium:
Es werden nur diejenigen Isoenergieschichten bestimmt und damit bestrahlt, welche laut dem ersten Durchlauf den größten Dosisbeitrag zur im Zielvolumen zu applizierenden Gesamtdosis im Vergleich zu den anderen Isoenergieschichten aufweisen.

6. Kriterium:
Es werden nur diejenigen Isoenergieschichten bestimmt und damit bestrahlt, welche laut dem ersten Durchlauf den größten Beitrag zu einer Zielfunktion, welche zur Bestimmung des Bestrahlungsplans eingesetzt wird, im Vergleich zu den anderen Isoenergieschichten aufweisen.

7. Kriterium:
Es werden nur diejenigen Isoenergieschichten bestimmt und damit bestrahlt, welche laut dem ersten Durchlauf den größten Dosiskompensationsfehler im Vergleich zu den anderen Isoenergieschichten aufweisen. Der Dosiskompensationsfehler gibt dabei ein Maß für einen Fehler an, welcher dadurch entsteht, dass eine Isoenergieschicht nicht bestrahlt wird, obwohl eine entsprechende Kompensation durch die bestrahlten Isoenergieschichten vorgenommen wird.

8. Kriterium:

Zur Bestimmung der Menge der zu bestrahlenden Isoenergieschichten wird gemäß dem 8. Kriterium von einer Menge von Isoenergieschichten ausgegangen, welche laut dem Bestrahlungsplan des ersten Durchlaufs zu bestrahlen sind. Dabei kann diese Menge, mit der die folgende Optimierung beginnt, auch keine Isoenergieschicht oder nur eine Isoenergieschicht umfassen. Es wird ein erster Zielfunktionswert anhand eines Bestrahlungsplans bestimmt, welcher auf der Basis der Menge von Isoenergieschichten optimiert bzw. erstellt wird, damit insbesondere die Ist-Dosis möglichst exakt der Soll-Dosis entspricht. Nun wird diejenige Isoenergieschicht gesucht, deren Hinzufügen zu der Menge nach einer Optimierung eines fiktiven Bestrahlungsplans zu einem zweiten Zielfunktionswert führt, welcher den größten Abstand zu dem ersten Zielfunktionswert aufweist (also zur größten Verbesserung führt).
Wenn dieser größte Abstand betragsmäßig größer als ein vorgegebener Abstandsschwellenwert ist, wird die entsprechende Isoenergieschicht der Menge hinzugefügt, so dass der erste Zielfunktionswert nun dem zweiten Zielfunktionswert entspricht, und das Vorgehen wird wiederholt. Die Menge der zu bestrahlenden Isoenergieschichten wird solange erweitert, bis keine Isoenergieschicht mehr gefunden wird, deren Hinzufügen zu der Menge nach einer Optimierung eines fiktiven Bestrahlungsplans zu einem (neuen) zweiten Zielfunktionswerts führt, welcher einen Abstand zu dem (neuen) ersten Zielfunktionswert aufweist, der größer als der vorgegebene Abstandsschwellenwert ist.

Das weitere erfindungsgemäße Verfahren ist quasi eine invers arbeitende Variante des erfindungsgemäßen Verfahrens, so dass das Ergebnis (insbesondere die Menge der bestrahlten Isoenergieschichten bzw. der nicht bestrahlten Isoenergieschichten) des weiteren erfindungsgemäßen Verfahrens dem Ergebnis des erfindungsgemäßen Verfahrens im Wesentlichen entspricht. Während das erfindungsgemäße Verfahren quasi mit allen Isoenergieschichten beginnt und dann nach gewissen Kriterien bestimmte Isoenergieschichten von der Bestrahlung ausschließt, bestimmt das weitere erfindungsgemäße Verfahren nach entsprechenden Kriterien die zu bestrahlenden Isoenergieschichten.
Wie bei dem erfindungsgemäßen Verfahren kann bei dem weiteren erfindungsgemäßen Verfahren ein Verhältnis der Anzahlen bzw. der Anteile entsprechend des jeweiligen Kriteriums der bestimmten Isoenergieschichten zu einer Summe der Anzahlen bzw. der Anteile entsprechend des jeweiligen Kriteriums aller Isoenergieschichten gebildet werden. Die bestimmten Isoenergieschichten werden dabei so gewählt, dass das derart bestimmte Verhältnis gerade über einem vorbestimmten Schwellenwert liegt, so dass das Entfernen auch nur einer der bestimmten Isoenergieschichten dazu führt, dass das Verhältnis unterhalb des vorbestimmten Schwellenwerts liegt.

Es ist ebenfalls möglich, die bestimmten Isoenergieschichten so zu wählen, dass die Summe der Anzahlen bzw. der Anteile entsprechend des jeweiligen Kriteriums der bestimmten Isoenergieschichten gerade oberhalb eines vorbestimmten Maximalwert liegt, so dass das Entfernen auch nur einer der bestimmten Isoenergieschichten dazu führt, dass die Summe unterhalb des vorbestimmten Maximalwert liegt.

Es kann auch eine bestimmte Bedingung für den Bestrahlungsplan vorgegeben werden. Es wird nun solange eine Isoenergieschicht den bestimmten Isoenergieschichten hinzugefügt, bis durch das Hinzufügen der letzten hinzugefügten Isoenergieschicht der damit bestimmte Bestrahlungsplan die Bedingung erfüllt.

Gemäß einer erfindungsgemäßen Ausführungsform kann das zur Bestimmung des Bestrahlungsplans eingesetzte Kriterium oder die zur Bestimmung des Bestrahlungsplans eingesetzten Kriterien von einem Winkelbereich abhängen, welchen zwei Felder, mit denen das Zielvolumen bestrahlt wird, zueinander ausbilden. Darüber hinaus kann das zur Bestimmung des Bestrahlungsplans eingesetzte Kriterium oder die zur Bestimmung des Bestrahlungsplans eingesetzten Kriterien (zusätzlich) von der Geometrie des Zielvolumens abhängen.

Beispielsweise ist es möglich, dass bestimmte Kriterien nur beim Einsatz von Gegenfeldern oder beim Einsatz von zwei Feldern, welche mindestens einen Winkel von 120° ausbilden, eingesetzt werden. Erfindungsgemäß ist es allerdings auch möglich, dass mit mehr als zwei Feldern bei der Bestrahlung gearbeitet wird.

Unter einem Feld wird dabei die Bestrahlung aus einer bestimmten Richtung verstanden. Bei einem Gegenfeld erfolgt die Bestrahlung aus zwei im Wesentlichen entgegengesetzten Richtungen. Daher können bei dem Einsatz von einem Gegenfeld beispielsweise die Isoenergieschichten mit der geringsten bzw. größten Energie nicht bestrahlt werden, da die entsprechenden nicht bestrahlten Bereiche von den Isoenergieschichten mit der größten bzw. geringsten Energie des jeweils anderen Felds abgedeckt werden können.

Bei einem konvexen Zielvolumen, welches in einen homogenen Bereich des Untersuchungsobjekts (z.B. eines Patienten) eingebettet ist, existieren beispielsweise Voxel im Zielvolumen, welche sowohl durch einen mit hoher Energie arbeitenden Teilchenstrahl des einen Feldes als auch durch einen mit vergleichsweise niedrigerer Energie arbeitenden Teilchenstrahl eines anderen Feldes mit Teilchen belegt werden.

Im Rahmen der vorliegenden Erfindung werden auch eine Vorrichtung und eine weitere Vorrichtung zur Bestimmung eines Bestrahlungsplans für eine Partikelbestrahlungsanlage bereitgestellt. Die Partikelbestrahlungsanlage bestrahlt dabei abhängig von dem bestimmten Bestrahlungsplan ein Zielvolumen innerhalb eines Untersuchungsobjekts mit einem Teilchenstrahl oder Partikelstrahl. Die Vorrichtungen umfassen jeweils Eingabemittel, Rechenmittel und Ausgabemittel. Mittels der Eingabemittel werden der jeweiligen Vorrichtung das Zielvolumen und eine vorbestimmte Dosisverteilung (Soll-Dosisverteilung) vorgegeben. Die Rechenmittel bestimmen den Bestrahlungsplan in einem ersten Durchlauf derart, dass die Teilchen des Teilchenstrahls entsprechend der vorbestimmten Dosisverteilung in dem Zielvolumen, welches mehrere Isoenergieschichten umfasst, abgegeben werden. Der Bestrahlungsplan wird jeweils mit den Ausgabemitteln ausgegeben.

Die Rechenmittel der erfindungsgemäßen Vorrichtung bestimmen den Bestrahlungsplan in einem zweiten Durchlauf zusätzlich unter der Bedingung, dass die Vorrichtung mindestens eine der Isoenergieschichten nicht bestrahlt. Dabei bestimmen die Rechenmittel die mindestens eine Isoenergieschicht (d.h. die n Isoenergieschichten) nach mindestens einem der Kriterien, welche den für das erfindungsgemäße Verfahren beschriebenen Kriterien entsprechen.

Dagegen bestimmen die Rechenmittel der weiteren erfindungsgemäßen Vorrichtung den Bestrahlungsplan in einem zweiten Durchlauf zusätzlich unter der Bedingung, dass nur bestimmte der Isoenergieschichten bestrahlt werden. Dabei bestimmen die Rechenmittel die bestimmten Isoenergieschichten nach mindestens einem der Kriterien, welche den für das weitere erfindungsgemäße Verfahren beschriebenen Kriterien entsprechen.

Die Vorteile der beiden erfindungsgemäßen Vorrichtungen entsprechen im Wesentlichen den Vorteilen der beiden erfindungsgemäßen Verfahren, welche vorab im Detail ausgeführt sind, so dass hier auf eine Wiederholung verzichtet wird.

Darüber hinaus stellt die vorliegende Erfindung eine Partikelbestrahlungsanlage mit einer erfindungsgemäßen Vorrichtung bereit.

Des Weiteren beschreibt die vorliegende Erfindung ein Computerprogrammprodukt, insbesondere eine Software, welche man in einen Speicher einer programmierbaren Steuereinrichtung bzw. Rechenmittel einer Partikelbestrahlungsanlage laden kann. Mit diesem Computerprogrammprodukt können alle oder verschiedene vorab beschriebene Ausführungsformen der erfindungsgemäßen Verfahren ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die entsprechenden Ausführungsformen der Verfahren zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen der erfindungsgemäßen Verfahren ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechenden Rechenmittel bzw. Steuereinrichtung zu laden ist.

Schließlich offenbart die vorliegende Erfindung einen elektronisch lesbaren Datenträger, z.B. eine DVD, ein Magnetband oder einen USB-Stick, auf welchem elektronisch lesbare Steuerinformationen, insbesondere Software (vgl. oben), gespeichert ist. Wenn diese Steuerinformationen (Software) von dem Datenträger gelesen und in Steuermittel bzw. eine Recheneinheit einer Partikelbestrahlungsanlage gespeichert werden, können alle erfindungsgemäßen Ausführungsformen der vorab beschriebenen Verfahren durchgeführt werden.

Die Auswahl des oder der Kriterien zur Bestimmung der nicht zu bestrahlenden Isoenergieschichten oder zur Bestimmung der zu bestrahlenden Isoenergieschichten kann dabei interaktiv oder mittels einer Konfigurationsseinsstellung von einem Benutzer vorgegeben werden.

Durch das gezielte Verringern der Isoenergieschichten (erste Variante) oder durch den gezielten Einsatz nur der wichtigsten Isoenergieschichten (zweite Variante) kann die Gesamtbestrahlungsdauer deutlich verkürzt werden, was zum größeren Komfort für den Patienten und einer Steigerung der Effektivität der Partikelbestrahlungsanlage führt. Die Einführung der vorliegenden Erfindung erfordert vorteilhafterweise keinen Eingriff in die Strahlenapplikation oder in den Beschleuniger selbst, da es sich lediglich um eine Softwareänderung handelt. Durch eine Wählbarkeit der Parameter (z.B. Auswahl der Kriterien) und eine Anzeige der Auswirkungen auf die Qualität des Bestrahlungsplans kann darüber hinaus vorteilhafterweise fallbezogen ein optimaler Kompromiss zwischen einer verkürzten Gesamtbestrahlungsdauer und der Qualität des Bestrahlungsplans gefunden werden.

Die vorliegende Erfindung ist insbesondere zur Erhöhung des Patientendurchsatzes bei der Partikeltherapie geeignet. Selbstverständlich ist die vorliegende Erfindung nicht auf diesen bevorzugten Anwendungsbereich eingeschränkt, da die vorliegende Erfindung prinzipiell überall dort eingesetzt werden kann, wo mit Teilchen oder Partikeln Energie bzw. eine Dosis in einem Zielvolumen appliziert wird.

Im Folgenden wird die vorliegende Erfindung anhand erfindungsgemäßer Ausführungsformen mit Bezug zu den Figuren im Detail beschrieben.

Fig. 1 stellt schematisch einen Überblick über den Aufbau einer Partikelbestrahlungsanlage dar.

In Fig. 2 ist schematisch dargestellt, wie ein Zielvolumen mittels einer Partikelbestrahlungsanlage bestrahlt wird.

Fig. 3 zeigt einen Ablaufplan einer ersten Variante der vorliegenden Erfindung.

Fig. 4 zeigt einen Ablaufplan einer zweiten Variante der vorliegenden Erfindung.

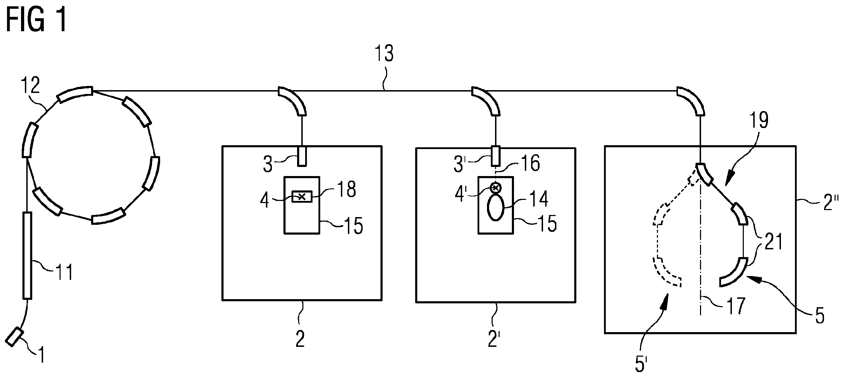

Die in Fig. 1 schematisch dargestellte Partikelbestrahlungsanlage 20 bestrahlt einen auf einer Positionierungsvorrichtung 15 (einem Tisch) liegenden Patienten 14 (siehe Bestrahlungsraum 2') mit einem Partikel bzw. Teilchen 16 umfassenden Strahl, welcher im Folgenden als Partikelstrahl bzw. Teilchenstrahl 16 bezeichnet wird. Mit einem solchen Teilchenstrahl 16 kann beispielsweise ein Tumor des Patienten 14 mit hochenergetischen Partikeln bestrahlt werden. Es ist allerdings auch möglich, die Partikelbestrahlungsanlage 20 zur Bestrahlung eines nicht-lebenden Objektes 18 einzusetzen, wie es im Bestrahlungsraum 2 am Beispiel eines Wasserphantoms 18 dargestellt ist.

Als Partikel bzw. Teilchen werden beispielsweise Protonen, Pionen, Heliumionen, Kohlenstoffionen, aber auch Ionen anderer Elemente eingesetzt. Dazu werden die entsprechenden Partikel in einer Partikelquelle bzw. Ionenquelle 1 erzeugt und in einem Vorbeschleuniger 11 (z. B. einem Linearbeschleuniger) auf ein erstes Energieniveau beschleunigt. Anschließend werden die Teilchen in einem Ringbeschleuniger 12 (z. B. einem Synchrotron oder Zyklotron) auf eine zur Bestrahlung benötigte Energie beschleunigt. Der aus dem Ringbeschleuniger 12 austretende Teilchenstrahl wird von einem Hochenergiestrahl-Transportsystem 13 in einen oder mehrere Bestrahlungsräume 2, 2', 2" transportiert, und dort zur Bestrahlung eines Zielvolumens eines Patienten 14 eingesetzt. Die Bestrahlung erfolgt von einer festen Richtung aus, so dass der zu bestrahlende Körper 14, 18 vorher raumfest mittels der Positionierungsvorrichtung 15 in dem Bestrahlungsraum 2, 2' angeordnet wird. Die Bestrahlungsräume 2, 2' werden daher auch als so genannte "fixed beam"-Räume bezeichnet. Dagegen existiert im Bestrahlungsraum 2" eine um eine Achse 17 beweglich angeordnete, insbesondere drehbare Gantry 19, mittels welcher der zu bestrahlende Körper von verschiedenen Richtungen aus (mit verschiedenen Feldern) bestrahlt werden kann. Dazu wird der Teilchenstrahl 16 mit Hilfe einer Strahlführung 21 der Gantry 19 entsprechend auf den zu bestrahlenden Körper gerichtet. In Fig. 1 sind zwei Positionen 5, 5' dargestellt, obwohl mehrere Positionen möglich sind.

In den Bestrahlungsräumen 2, 2' tritt der Teilchenstrahl 16 aus einem Strahlauslass 3, 3' aus und trifft auf den Körper 14 bzw. 18, in welchem sich das zu bestrahlende Zielvolumen befindet. Das Zielvolumen liegt dabei normalerweise in dem Isozentrum 4, 4' des jeweiligen Bestrahlungsraums 2, 2'.

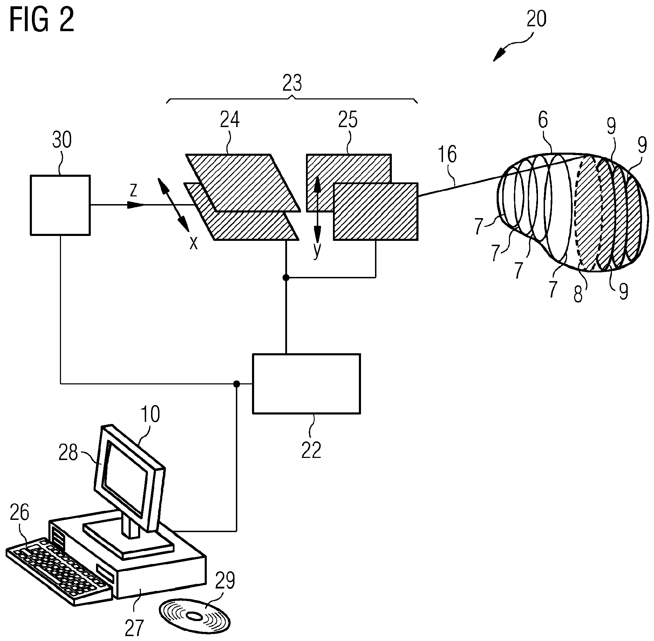

In Fig. 2 ist schematisch ein Zielvolumen 6 dargestellt, welches von einem mittels einer Partikelbestrahlungsanlage 20 erzeugten Teilchenstrahl 16 bestrahlt wird. Die Partikelbestrahlungsanlage 20 umfasst neben einer Bestrahlungsplanungsvorrichtung 10 eine Strahlerzeugungsvorrichtung 30, eine Rasterscan-Einrichtung 23 und eine Steuerung 22 für die Rasterscan-Einrichtung 23. Die Rasterscan-Einrichtung 23 weist wiederum eine erste Partikelablenkung 24 und eine zweite Partikelablenkung 25 auf, welche jeweils insbesondere Magnete umfassen. Mit Hilfe der beiden Partikelablenkungen 24, 25 kann der Teilchenstrahl 16 sowohl horizontal als auch vertikal abgelenkt werden, was durch die zueinander senkrecht stehenden Pfeile x, y dargestellt ist. Daher ist die Rasterscan-Einrichtung 23 in der Lage, den Teilchenstrahl 16 auf einen beliebigen Punkt $(x_i, y_i)$ einer Fläche innerhalb der x-y-Ebene zu lenken. Jeder dieser Punkte wird zusammen mit der jeweils eingesetzten Partikelenergie als Scan Spot, Rasterpunkt oder Abtastpunkt bezeichnet.

Demnach ist ein Rasterpunkt zum einen durch die Ausrichtung des Teilchenstrahls 16 (x- bzw. y-Richtung) und zum anderen durch seine Partikelenergie bestimmt. Mit anderen Worten existieren für bestimmte x- und y-Koordinaten mehrere Rasterpunkte mit unterschiedlichen Partikelenergien. Die Partikelenergie bestimmt dabei quasi (unter Berücksichtigung des durchstrahlten Körpers, welcher z.B. durch eine CT-Aufnahme ermittelt wird) die Koordinate in der z-Richtung (senkrecht auf der x- bzw. y-Achse), wobei im Allgemeinen gilt, dass die z-Position des Bragg-Peaks umso weiter in Richtung des Teilchenstrahls 16 innerhalb des Zielvolumens 6 liegt, umso größer die Partikelenergie ist. Da allerdings die Eindringtiefe vom Gewebe bzw. Material abhängig ist, welches der Teilchenstrahl 16 durchläuft, gilt obiger Zusammenhang nur für dieselben x- bzw. y-Positionen exakt.

Unter dem Bragg-Peak wird dabei derjenige Punkt bzw. Bereich verstanden, in welchem der Teilchenstrahl entlang seiner Bahn den größten Anteil seiner Dosis appliziert.

Das mit dem Teilchenstrahl 16 zu bestrahlende Zielvolumen 6 wird dabei in Form von Isoenergieschichten 7-9 bestrahlt. In den Rasterpunkten derselben Isoenergieschicht 7-9 werden dabei jeweils Partikel mit derselben Energie appliziert. Unter der Voraussetzung, dass der Teilchenstrahl 16 auf seinem Weg zu der entsprechenden Isoenergieschicht 7-9 ein homogenes Volumen durchläuft, liegen die Isoenergieschichten 7-9 senkrecht zur z-Achse, wie es vereinfachend in Fig. 2 dargestellt ist.

Zur Einstellung des Teilchenstrahls 16 auf eine entsprechende Isoenergieschicht 7-9 (d.h. zur Positionierung des Bragg-Peaks auf einer Isoenergieschicht 7-9) wird den Partikeln des Teilchenstrahls 16 jeweils eine entsprechende Anfangsenergie zugewiesen, indem die Partikel auf eine dieser Anfangsenergie entsprechende Geschwindigkeit beschleunigt werden. Die Anfangsenergie beschreibt dabei die Energie eines Partikels, welches dieses Partikel vor dem Auftreffen auf das Objekt 14 bzw. 18 aufweist. Zur Bestrahlung derjenigen Isoenergieschicht 7, welche dem Strahlauslass 3, 3' am nächsten liegt (am weitesten links in Fig. 2), werden dazu Partikel mit der niedrigsten Energie eingesetzt, wohingegen zur Bestrahlung derjenigen Isoenergieschicht 9, welche von dem Strahlauslass 3, 3' am weitesten entfernt angeordnet ist (am weitesten rechts in Fig. 2), die Partikel mit der höchsten Energie verwendet werden.

Zur Bestrahlung des gesamten Zielvolumens 6 werden die Isoenergieschichten 7-9 nacheinander bestrahlt, wobei in der Regel mit der Isoenergieschicht 9, welche am weitesten vom Strahlauslass 3, 3' entfernt ist, begonnen wird und dann mit der jeweils benachbarten Isoenergieschicht fortgefahren wird. Um bestimmte Rasterpunkte innerhalb derselben Isoenergieschicht 7-9 mit unterschiedlichen Energien zu bestrahlen, wird insbesondere die Zeitspanne variiert, während welcher der entsprechende Rasterpunkt von dem Teilchenstrahl 16 bestrahlt wird. Je länger der entsprechende Rasterpunkt von dem Teilchenstrahl 16 bestrahlt wird, desto mehr Energie (höhere Dosis) wird in dem entsprechenden Rasterpunkt deponiert.

Bei dem in Fig. 2 dargestellten Zielvolumen 6 wird aktuell die Isoenergieschicht 8 von dem Teilchenstrahl 16 bestrahlt, während die drei Isoenergieschichten 9 bereits bestrahlt worden sind und die weiter links (in Fig. 2) liegenden vier Isoenergieschichten 7 noch auf ihre Bestrahlung warten.

Bevor das Zielvolumen 6 bestrahlt wird, wird ein Bestrahlungsplan erstellt, mittels welchem das Scannen bzw. Abtasten des Zielvolumens 6 mit dem Teilchenstrahl 16 erfolgt. Der Bestrahlungsplan bestimmt dabei insbesondere Steuerparameter zur Besteuerung der Partikelbestrahlungsanlage 20. Die Erstellung des Bestrahlungsplans wird dabei mit Hilfe einer Bestrahlungsplanungsvorrichtung 10 (beispielsweise einem PC) durchgeführt.

Zur Durchführung der eigentlichen Bestrahlung wird der Bestrahlungsplan von der Bestrahlungsplanungsvorrichtung 10 an die Strahlerzeugungsvorrichtung 30 und die Steuerung 22 der Rasterscan-Einrichtung 23 weitergeleitet. In Fig. 2 ist die Bestrahlungsplanungsvorrichtung 10 quasi als Bestandteil der Partikelbestrahlungsanlage 20 dargestellt. Natürlich ist es ebenso gut erfindungsgemäß möglich, dass der von der Bestrahlungsplanungsvorrichtung 10 erstellte Bestrahlungsplan auf einen Datenträger 29 geladen wird, über welchen dann der Bestrahlungsplan in die Partikelbestrahlungsanlage 20 geladen wird. In diesem Fall müssen die Bestrahlungsplanungsvorrichtung 10 und die Partikelbestrahlungsanlage 20 nicht miteinander kommunikationstechnisch verbunden sein. Darüber hinaus kann zwischen der Erstellung des Bestrahlungsplans und der anhand des Bestrahlungsplans durchgeführten Bestrahlung ein gewisser Zeitraum, beispielsweise mehrere Tage, liegen.

Zur Erstellung des Bestrahlungsplans benötigt die Bestrahlungsplanungsvorrichtung 10 die Lage und die Ausmaße des zu bestrahlenden Zielvolumens 6 (z. B. eines zu bestrahlenden Tumors). Darüber hinaus wird bei der Bestrahlung eines Patienten 14 die Beschaffenheit des Gewebes benötigt, welches von dem Teilchenstrahl 16 auf dem Weg zu dem Zielvolumen 6 durchstrahlt wird. Diese Informationen können beispielsweise mittels eines Computer- oder Magnetresonanztomographen ermittelt werden und dann über entsprechende Eingabemittel 26 an die Bestrahlungsplanungsvorrichtung 10 übermittelt werden. Die Bestrahlungsplanungsvorrichtung 10 bestimmt mit Hilfe ihrer Rechenmittel 27 ausgehend von diesen Informationen und einer vorbestimmten Dosisverteilung (Soll-Dosisverteilung) den Bestrahlungsplan. Der Bestrahlungsplan gibt dabei insbesondere an, wie viele Partikel einer bestimmten Energie an einem Rasterpunkt zu applizieren sind.

Ein Patient muss während der Bestrahlung fixiert werden, um eine Bewegung des Zielvolumens 6 möglichst auszuschließen. Aus diesem Grund sollte die Bestrahlungsdauer möglichst kurz gehalten werden. Darüber hinaus ermöglicht eine kurze Bestrahlungsdauer vorteilhafterweise einen höheren Patientendurchsatz. Auf der anderen Seite sollte die Dosisverteilung gemäß Bestrahlungsplan möglichst gut der Soll-Dosisverteilung entsprechen. Indem erfindungsgemäß die Anzahl der bestrahlten Isoenergieschichten im Vergleich zum Stand der Technik kleiner ist, weist ein erfindungsgemäß erstellter Bestrahlungsplan eine geringere Gesamtbestrahlungsdauer auf.

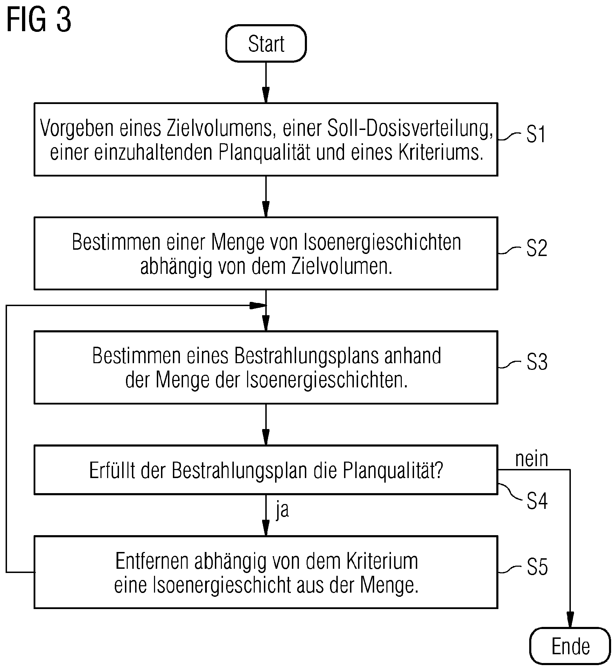

In Fig. 3 ist ein Ablaufplan einer ersten Variante der vorliegenden Erfindung dargestellt.

In einem ersten Schritt S1 werden zur Erstellung eines Bestrahlungsplans ein Zielvolumen, eine Soll-Dosisverteilung, eine einzuhaltende Planqualität des zu erstellenden Bestrahlungsplans und ein Kriterium zur Auswahl von Isoenergieschichten vorgegeben.

Die Planqualität umfasst insbesondere die Qualität der Dosisverteilung (d.h. die Qualität, mit welcher die Dosis des Teilchenstrahls anhand des Bestrahlungsplans appliziert wird). Diese Qualität der Dosisverteilung kann dabei beispielsweise an der Einhaltung bestimmter Toleranzgrenzen von Risikoorganen, anhand von Dosis-Volumen-Histogrammen, anhand der Einhaltung von Toleranzgrenzen hinsichtlich der Dosisverteilung innerhalb des Zielvolumens ("dose constraints of planning target volume") und/oder anhand der Übereinstimmung zwischen tatsächlicher Dosisverteilung und Soll-Dosisverteilung (insbesondere müssen die absoluten Werte der tatsächlichen Dosisverteilung (z.B. gemäß Bestrahlungsplan) den absoluten Werten der Soll-Dosisverteilung entsprechen) bestimmt werden.

In einem zweiten Schritt S2 werden in Abhängigkeit von dem vorgegebenen Zielvolumen die Isoenergieschichten bestimmt, welche anfänglich die Menge der Isoenergieschichten definieren.

In einem dritten Schritt S3 wird der (vorläufige) Bestrahlungsplan abhängig von der vorgegebenen Soll-Dosisverteilung und der Menge der Isoenergieschichten erstellt. Dabei legt der Bestrahlungsplan unter anderem fest, wie viele Rasterpunkte jede Isoenergieschicht aus der Menge aufweist und mit wie vielen Partikeln bzw. Teilchen jeder dieser Rasterpunkte zu bestrahlen ist, um durch diese Bestrahlung neben anderen Randbedingungen der Soll-Dosisverteilung möglichst nahe zu kommen. Daher legt der Bestrahlungsplan für jede Isoenergieschicht der Menge auch die Gesamtpartikelanzahl fest.

Im vierten Schritt S4 wird nun überprüft, ob der im Schritt S3 erstellte Bestrahlungsplan die vorgegebene Planqualität erfüllt.

Wenn dies der Fall ist, wird im fünften Schritt S5 abhängig von dem vorgegebenen Kriterium eine Isoenergieschicht aus der Menge entfernt. Bei diesem Kriterium kann es sich beispielsweise um die Rasterpunktanzahl oder um die Gesamtpartikelanzahl handeln, so dass diejenige Isoenergieschicht aus der Menge entfernt wird, welche nach dem aktuellen Bestrahlungsplan die kleinste Rasterpunktanzahl oder die kleinste Gesamtpartikelanzahl aufweist.

Das Verfahren verzweigt anschließend wieder zurück zum Schritt S3, in welchem anhand der nun reduzierten Menge der Isoenergieschichten erneut ein (neuer) Bestrahlungsplan erstellt wird. Dieses Durchführen der Schritte S3 bis S5 wird so lange fortgesetzt, bis der Bestrahlungsplan die vorgegebene Planqualität nicht mehr erfüllt. In diesem Fall kann entweder der aktuelle Bestrahlungsplan als endgültiger Bestrahlungsplan, mit welchem schließlich die Bestrahlung durchgeführt wird, eingesetzt werden oder die zuletzt entfernte Isoenergieschicht wird in einem nicht dargestellten Schritt wieder zur Menge der Isoenergieschichten hinzugefügt, und es wird abhängig von dieser Menge ein Bestrahlungsplan erstellt, welcher dann zur Bestrahlung eingesetzt wird. Natürlich ist es auch möglich, dass jeweils der vor dem aktuellen Bestrahlungsplan bestimmte Bestrahlungsplan abgespeichert ist, so dass dieser als endgültiger Bestrahlungsplan sofort verwendet werden kann, sollte der aktuelle bzw. zuletzt bestimmte Bestrahlungsplan die Planqualität nicht erfüllen.

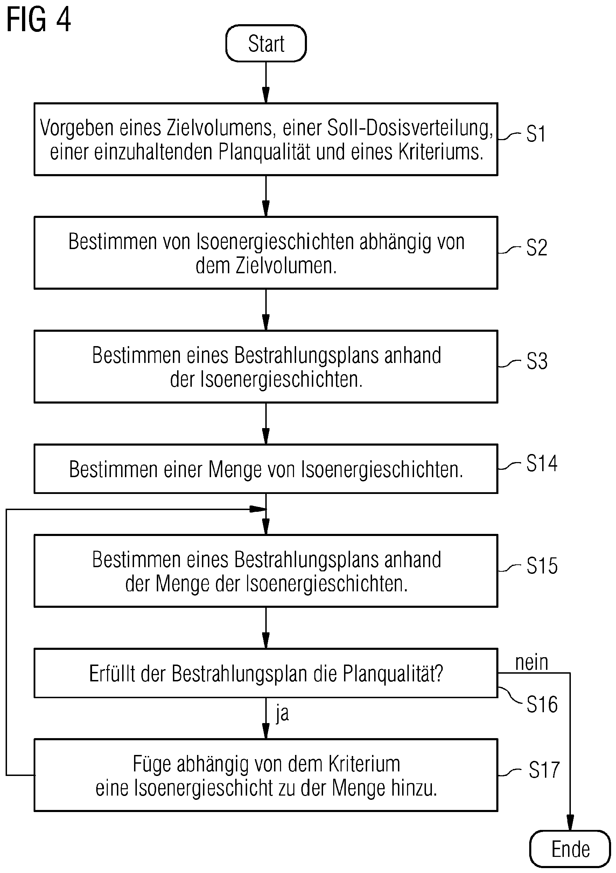

In Fig. 4 ist ein Ablauf einer zweiten Variante der vorliegenden Erfindung dargestellt.

Man erkennt, dass der Ablauf der zweiten Variante Ähnlichkeit mit dem Ablauf der ersten Variante aufweist. Während laut der ersten Variante aus der Menge der Isoenergieschichten so lange nach bestimmten Kriterien Isoenergieschichten entfernt werden, bis die Planqualität gerade noch akzeptabel ist, werden laut der zweiten Variante so lange nach den bestimmten Kriterien der Menge Isoenergieschichten hinzugefügt, bis die Planqualität akzeptabel ist.

Auch bei der zweiten Variante werden im ersten Schritt S1 ein Zielvolumen, eine Soll-Dosisverteilung, eine einzuhaltende Planqualität und ein Kriterium vorgegeben.

Wie bei der ersten Variante werden bei der zweiten Variante im zweiten Schritt S2 abhängig von dem Zielvolumen die Isoenergieschichten bestimmt, welche wie bei der ersten Variante derart bestimmt werden, dass das Zielvolumen möglichst vollständig von den Isoenergieschichten durchzogen wird.

Im dritten Schritt S3 wird anhand dieser derart bestimmten Isoenergieschichten ein Bestrahlungsplan bestimmt.

Im vierten Schritt S14 wird nun eine Menge von Isoenergieschichten bestimmt. Bei der Bestimmung dieser Menge kann das vorgegebene Kriterium eingesetzt werden, so dass beispielsweise diejenigen Isoenergieschichten gewählt werden, welche die meisten Rasterpunkte aufweisen oder die größte Gesamtpartikelanzahl (laut dem in Schritt S3 erstellten Bestrahlungsplan) besitzen. Es ist allerdings auch möglich, dass im Schritt S14 eine leere Menge bestimmt wird oder dass nur eine, nämlich diejenige Isoenergieschicht als Menge bestimmt wird, welche nach dem vorgegebenen Kriterium als am besten erscheint.

Abhängig von dieser bestimmten Menge von Isoenergieschichten wird im Schritt S15 ein Bestrahlungsplan bestimmt, dessen Planqualität im Schritt S16 überprüft wird. Wenn die Planqualität des Bestrahlungsplans nicht erfüllt ist, was beim ersten Durchlauf eigentlich immer der Fall sein sollte, wird im Schritt S17 abhängig von dem vorgegebenen Kriterium eine noch nicht in der Menge vorhandene Isoenergieschicht der Menge hinzugefügt. Beispielsweise kann diejenige Isoenergieschicht hinzugefügt werden, welche von den noch nicht in der Menge befindlichen Isoenergieschichten die meisten Rasterpunkte oder die größte Gesamtpartikelanzahl (laut dem in Schritt S3 erstellten Bestrahlungsplan) aufweist. Anschließend verzweigt der Ablauf der zweiten Variante wieder zurück zu dem Schritt S15, in welchem nun abhängig von der erweiterten Menge der Isoenergieschichten ein besserer Bestrahlungsplan bestimmt wird.

Sobald im Schritt S16 erfasst wird, dass die Menge der Isoenergieschichten ausreicht, so dass der damit erstellte Bestrahlungsplan die vorgegebene Planqualität erfüllt, endet das Verfahren, wobei der dann aktuelle Bestrahlungsplan dem endgültigen Bestrahlungsplan entspricht, mit welchem schließlich die Bestrahlung vorgenommen wird.

## Patentansprüche

1.  Verfahren zum Bestimmen eines Bestrahlungsplans für eine Partikelbestrahlungsanlage (20),
    wobei mit der Partikelbestrahlungsanlage (20) abhängig von dem Bestrahlungsplan ein Zielvolumen (6) innerhalb eines Untersuchungsobjekts (14; 18) mit einem Teilchenstrahl (16) bestrahlt wird,
    wobei das Zielvolumen (6) und eine vorbestimmte Dosisverteilung vorgegeben werden,
    wobei der Bestrahlungsplan in einem ersten Durchlauf bestimmt wird, um den Teilchenstrahl (16) gemäß der vorbestimmten Dosisverteilung in dem Zielvolumen (6), welches mehrere Isoenergieschichten (7-9) umfasst, zu applizieren,
    **dadurch gekennzeichnet,**
    **dass** der Bestrahlungsplan in einem zweiten Durchlauf zusätzlich unter der Bedingung bestimmt wird, dass nur bestimmte der Isoenergieschichten bestrahlt werden, und
    **dass** die bestimmten Isoenergieschichten nach zumindest einem von folgenden Kriterien bestimmt werden:

    • es werden nur die bestimmten Isoenergieschichten bestrahlt, zu deren Bestrahlung der Teilchenstrahl eine Energie aufweist, welche oberhalb einer minimalen Grenzenergie liegt,
    • es werden nur die bestimmten Isoenergieschichten bestrahlt, zu deren Bestrahlung der Teilchenstrahl eine Energie aufweist, welche unterhalb einer maximalen Grenzenergie liegt,
    • es werden nur die bestimmten Isoenergieschichten bestrahlt, welche laut dem Bestrahlungsplan nach dem ersten Durchlauf die größte Anzahl von Rasterpunkten der Isoenergieschichten (7-9) aufweisen,
    • es werden nur die bestimmten Isoenergieschichten bestrahlt, welche laut dem Bestrahlungsplan nach dem ersten Durchlauf die größte Gesamtpartikelanzahl der Isoenergieschichten (7-9) aufweisen,
    • es werden nur die bestimmten Isoenergieschichten bestrahlt, welche laut dem Bestrahlungsplan nach dem ersten Durchlauf den größten Dosisbeitrag zur im Zielvolumen zu applizierenden Gesamtdosis aufweisen,
    • es werden nur die bestimmten Isoenergieschichten bestrahlt, welche laut dem Bestrahlungsplan nach dem ersten Durchlauf den größten Beitrag zu einer Zielfunktion, welche zur Bestimmung des Bestrahlungsplans berechnet wird, aufweisen,
    • es werden nur die bestimmten Isoenergieschichten bestrahlt, welche laut dem Bestrahlungsplan nach dem ersten Durchlauf den größten Dosiskompensationsfehler aufweisen, wobei der Dosiskompensationsfehler einen Fehler angibt, welcher durch das Nicht-Bestrahlen der bestimmten Isoenergieschichten trotz entsprechender Kompensation durch die bestrahlten Isoenergieschichten auftritt,
    • die bestimmten Isoenergieschichten werden ausgehend von einer Menge bestimmt, welche gemäß dem Bestrahlungsplan des ersten Durchlaufs zu bestrahlen sind,
    wobei jeweils ein erster Zielfunktionswert anhand eines Bestrahlungsplans erstellt wird, der unter der Voraussetzung erstellt wird, dass nur die Menge der Isoenergieschichten bestrahlt wird,
    wobei jeweils diejenige Isoenergieschicht zu der Menge hinzugefügt wird, welche gegenüber dem ersten Zielfunktionswert einen zweiten Zielfunktionswert am stärksten ändert, welcher anhand eines Bestrahlungsplans bestimmt wird, der unter der Voraussetzung erstellt wird, dass nur diejenige Isoenergieschicht zusätzlich zu der Menge bestrahlt wird,
    wobei solange eine weitere Isoenergieschicht der Menge hinzugefügt wird, solange die Änderung des zweiten Zielfunktionswerts gegenüber dem ersten Zielfunktionswert größer als ein vorgegebener Abstandsschwellenwert ist,
    • es werden nur die bestimmten Isoenergieschichten bestrahlt, welche gemäß einer beliebigen Kombination der beschriebenen Kriterien bestimmt werden.

2.  Verfahren nach Anspruch 1,
    **dadurch gekennzeichnet,**
    **dass** ein Verhältnis der Anzahlen bzw. der Anteile entsprechend des jeweiligen Kriteriums der bestimmten Isoenergieschichten zu einer Summe der Anzahlen bzw. der Anteile entsprechend des jeweiligen Kriteriums aller Isoenergieschichten gebildet wird,

**dass** die bestimmten Isoenergieschichten so gewählt werden, dass das Verhältnis über einem vorbestimmten Schwellenwert liegt, und

**dass** ein Entfernen einer der bestimmten Isoenergieschichten dazu führt, dass das Verhältnis unterhalb des vorbestimmten Schwellenwerts liegt.

3. Verfahren nach Anspruch 1,
   **dadurch gekennzeichnet,**
   **dass** eine Summe der Anzahlen bzw. der Anteile entsprechend des jeweiligen Kriteriums der bestimmten Isoenergieschichten gebildet wird,
   **dass** die bestimmten Isoenergieschichten so gewählt werden, dass die Summe oberhalb eines vorbestimmten Maximalwerts liegt, und
   **dass** ein Entfernen einer der bestimmten Isoenergieschichten dazu führt, dass die Summe unterhalb des vorbestimmten Maximalwerts liegt.

4. Verfahren nach einem der vorhergehenden Ansprüche,
   **dadurch gekennzeichnet,**
   **dass** eine von dem Bestrahlungsplan einzuhaltende Bedingung vorgegeben wird, und
   **dass** so lange mindestens eine Isoenergieschicht gemäß einem der Kriterien den bestimmten Isoenergieschichten hinzugefügt wird, bis der Bestrahlungsplan die Bedingung einhält.

5. Verfahren nach einem der vorhergehenden Ansprüche,
   **dadurch gekennzeichnet,**
   **dass** das mindestens eine Kriterium von einem Winkelbereich abhängt, welchen zwei Felder, mit denen das Zielvolumen (6) bestrahlt wird, zueinander ausbilden, und/oder dass das mindestens eine Kriterium von der Geometrie des Zielvolumens (6) abhängt.

6. Verfahren nach einem der vorhergehenden Ansprüche,
   **dadurch gekennzeichnet,**
   **dass** die Zielfunktion einen Bestrafungsterm umfasst,
   **dass** der Bestrafungsterm umso größer ist, je mehr der Isoenergieschichten (7-9) bestrahlt werden.

7. Vorrichtung zum Bestimmen eines Bestrahlungsplans für eine Partikelbestrahlungsanlage (20),
   wobei mit der Partikelbestrahlungsanlage (20) abhängig von dem Bestrahlungsplan ein Zielvolumen (6) innerhalb eines Untersuchungsobjekts (14; 18) mit einem Teilchenstrahl (16) bestrahlt wird,
   wobei die Vorrichtung (10) Eingabemittel (26), Rechenmittel (27) und Ausgabemittel (28) umfasst,
   wobei der Vorrichtung (10) mittels der Eingabemittel (26) das Zielvolumen (6) und eine vorbestimmte Dosisverteilung vorgebbar sind,
   wobei die Rechenmittel (27) den Bestrahlungsplan in einem ersten Durchlauf bestimmen, um den Teilchenstrahl (16) gemäß der vorbestimmten Dosisverteilung in dem Zielvolumen (6), welches mehrere Isoenergieschichten (7-9) umfasst, zu applizieren, und
   wobei die Ausgabemittel (28) den Bestrahlungsplan ausgeben,
   **dadurch gekennzeichnet,**
   **dass** der Vorrichtung (10) mittels der Eingabemittel (26) mindestens ein Kriterium für die Isoenergieschichten (7-9) vorgebbar ist,
   **dass** die Rechenmittel (27) den Bestrahlungsplan in einem zweiten Durchlauf zusätzlich unter der Bedingung bestimmen, dass die Vorrichtung (10) nur bestimmte der Isoenergieschichten bestrahlen lässt, und
   **dass** die Rechenmittel (27) die bestimmten Isoenergieschichten nach dem mindestens einen Kriterium, welches ausgewählt ist aus folgender Menge, bestimmen:

   • die Vorrichtung (10) bestrahlt nur die bestimmten Isoenergieschichten, zu deren Bestrahlung der Teilchenstrahl eine Energie aufweist, welche oberhalb einer minimalen Grenzenergie liegt,
   • die Vorrichtung (10) bestrahlt nur die bestimmten Isoenergieschichten, zu deren Bestrahlung der Teilchenstrahl eine Energie aufweist, welche unterhalb einer maximalen Grenzenergie liegt,
   • die Vorrichtung (10) bestrahlt nur die bestimmten Isoenergieschichten, welche laut dem Bestrahlungsplan nach dem ersten Durchlauf die größte Anzahl von Rasterpunkten der Isoenergieschichten (7-9) aufweisen,
   • die Vorrichtung (10) bestrahlt nur die bestimmten Isoenergieschichten, welche laut dem Bestrahlungsplan nach dem ersten Durchlauf die größte Gesamtpartikelanzahl der Isoenergieschichten (7-9) aufweist,

• die Vorrichtung (10) bestrahlt nur die bestimmten Isoenergieschichten, welche laut dem Bestrahlungsplan nach dem ersten Durchlauf den größten Dosisbeitrag zur im Zielvolumen zu applizierenden Gesamtdosis aufweist,

• die Vorrichtung (10) bestrahlt nur die bestimmten Isoenergieschichten, welche laut dem Bestrahlungsplan nach dem ersten Durchlauf den größten Beitrag zu einer Zielfunktion, welche zur Bestimmung des Bestrahlungsplans berechnet wird, aufweist,

• die Vorrichtung (10) bestrahlt nur die bestimmten Isoenergieschichten, welche laut dem Bestrahlungsplan nach dem ersten Durchlauf den größten Dosiskompensationsfehler aufweisen, wobei der Dosiskompensationsfehler einen Fehler angibt, welcher durch das Nicht-Bestrahlen der bestimmten Isoenergieschichten trotz entsprechender Kompensation durch die bestrahlten Isoenergieschichten auftritt,

• die Vorrichtung (10) bestrahlt nur die bestimmten Isoenergieschichten, welche ausgehend von einer Menge bestimmt werden, die gemäß dem Bestrahlungsplan des ersten Durchlaufs zu bestrahlen sind,

wobei jeweils ein erster Zielfunktionswert anhand eines Bestrahlungsplans erstellt wird, der unter der Voraussetzung erstellt wird, dass nur die Menge der Isoenergieschichten bestrahlt wird,

wobei jeweils diejenige Isoenergieschicht zu der Menge hinzugefügt wird, welche gegenüber dem ersten Zielfunktionswert einen zweiten Zielfunktionswert am stärksten ändert, welcher anhand eines Bestrahlungsplans bestimmt wird, der unter der Voraussetzung erstellt wird, dass nur diejenige Isoenergieschicht zusätzlich zu der Menge bestrahlt wird,

wobei solange eine weitere Isoenergieschicht der Menge hinzugefügt wird, solange die Änderung des zweiten Zielfunktionswerts gegenüber dem ersten Zielfunktionswert größer als ein vorgegebener Abstandsschwellenwert ist,

• die Vorrichtung (10) bestrahlt nur die bestimmten Isoenergieschichten, welche gemäß einer beliebigen Kombination der beschriebenen Kriterien bestimmt werden.

8. Vorrichtung nach Anspruch 7,
   **dadurch gekennzeichnet,**
   **dass** die Vorrichtung (10) zur Durchführung des Verfahrens nach einem der Ansprüche 1-6 ausgestaltet ist.

9. Partikelbestrahlungsanlage mit einer Vorrichtung (10) nach Anspruch 7 oder 8.

10. Computerprogrammprodukt, welches ein Programm umfasst und direkt in einen Speicher einer programmierbaren Steuereinrichtung einer Partikelbestrahlungsanlage (20) ladbar ist, mit Programm-Mitteln, um alle Schritte des Verfahrens nach einem der Ansprüche 1-6 auszuführen, wenn das Programm in der Steuereinrichtung der Partikelbestrahlungsanlage (20) ausgeführt wird.

11. Elektronisch lesbarer Datenträger mit darauf gespeicherten elektronisch lesbaren Steuerinformationen, welche derart ausgestaltet sind, dass sie bei Verwendung des Datenträgers (29) in einer Steuereinrichtung einer Partikelbestrahlungsanläge (20) das Verfahren nach einem der Ansprüche 1-6 durchführen.

## Claims

1. Computer-implemented method for determining an irradiation plan for a particle irradiation unit (20),
   wherein the particle irradiation unit (20) can be used to irradiate a target volume (6) within an examination object (14; 18) with a particle beam (16) depending on the irradiation plan,
   wherein the target volume (6) and a predetermined dose distribution are prescribed,
   wherein the irradiation plan is determined in a first calculation pass so that the particle beam (16) can be applied in accordance with the predetermined dose distribution in the target volume (6) which comprises a plurality of isoenergy layers (7-9),
   **characterized**
   **in that** the irradiation plan is determined in a second calculation pass with the additional condition that only specific ones of the isoenergy layers are irradiated, and
   **in that** the specific isoenergy layers are determined according to at least one of the following criteria:

   • only the specific isoenergy layers where the particle beam for the irradiation of said layers has an energy lying above a minimum peak energy are irradiated,
   • only the specific isoenergy layers where the particle beam for the irradiation of said layers has an energy lying below a maximum peak energy are irradiated,

• only the specific isoenergy layers which, according to the irradiation plan according to the first calculation pass, have the largest number of raster points of the isoenergy layers (7-9) are irradiated,

• only the specific isoenergy layers which, according to the irradiation plan according to the first calculation pass, have the largest overall number of particles of the isoenergy layers (7-9) are irradiated,

• only the specific isoenergy layers which, according to the irradiation plan according to the first calculation pass, make the largest dose contribution to the overall dose to be applied in the target volume are irradiated,

• only the specific isoenergy layers which, according to the irradiation plan according to the first calculation pass, make the largest contribution to a target function, which is calculated for determining the irradiation plan, are irradiated,

• only the specific isoenergy layers which, according to the irradiation plan according to the first calculation pass, have the largest dose compensation error are irradiated, wherein the dose compensation error specifies an error which occurs as a result of the non-irradiation of the specific isoenergy layers despite corresponding compensation by the irradiated isoenergy layers,

• the specific isoenergy layers are determined proceeding from a set which, in accordance with the irradiation plan of the first pass, are to be irradiated,

wherein respectively a first target function value is generated on the basis of an irradiation plan which is generated under the assumption that only the set of the isoenergy layers is irradiated,

wherein in each case that isoenergy layer is added to the set which, in relation to the first target function value, modifies a second target function value to the greatest extent, which second target function value is determined on the basis of an irradiation plan generated under the assumption that only that isoenergy layer is irradiated in addition to the set,

wherein a further isoenergy layer is added to the set for as long as the change of the second target function value in relation to the first target function value is greater than a predetermined distance threshold,

• only the specific isoenergy layers which are determined in accordance with any combination of the described criteria are irradiated.

2. Method according to Claim 1,
**characterized**
**in that** a ratio is formed between the numbers or the proportions, in accordance with the respective criterion, of the specific isoenergy layers and a sum of the numbers or the proportions, in accordance with the respective criterion, of all isoenergy layers,
**in that** the specific isoenergy layers are selected in such a way that the ratio lies above a predetermined threshold, and in that a removal of one of the specific isoenergy layers leads to the ratio lying below the predetermined threshold.

3. Method according to Claim 1,
**characterized**
**in that** a sum is formed of the numbers or the proportions, in accordance with the respective criterion, of the specific isoenergy layers,
**in that** the specific isoenergy layers are selected in such a way that the sum lies above a predetermined maximum value, and in that a removal of one of the specific isoenergy layers leads to the sum lying below the predetermined maximum value.

4. Method according to one of the preceding claims,
**characterized**
**in that** a condition to be complied with by the irradiation plan is prescribed, and
**in that** at least one isoenergy layer is added to the specific isoenergy layers in accordance with one of the criteria until the irradiation plan complies with the condition.

5. Method according to one of the preceding claims,
**characterized**
**in that** the at least one criterion depends on an angle range which is formed between two fields by means of which the target volume (6) is irradiated, and/or
**in that** the at least one criterion depends on the geometry of the target volume (6).

6. Method according to one of the preceding claims,
**characterized**
**in that** the target function contains a penalty term,

**in that** the penalty term becomes larger as the number of irradiated isoenergy layers (7-9) increases.

7. Device for determining an irradiation plan for a particle irradiation unit (20),
wherein the particle irradiation unit (20) is used to irradiate a target volume (6) within an examination object (14; 18) with a particle beam (16) depending on the irradiation plan,
wherein the device (10) comprises input means (26), computing means (27) and output means (28),
wherein the target volume (6) and a predetermined dose distribution are prescribable for the device (10) by means of the input means (26),
wherein the computing means (27) determine the irradiation plan in a first calculation pass in order to apply the particle beam (16) in the target volume (6) which comprises a plurality of isoenergy layers (7-9), in accordance with the predetermined dose distribution, and
wherein the output means (28) output the irradiation plan,
**characterized**
**in that** at least one criterion for the isoenergy layers (7-9) is prescribable for the device (10) by means of the input means (26),
**in that** the computing means (27) determine the irradiation plan in a second calculation pass with the additional condition that the device (10) only causes specific ones of the isoenergy layers to be irradiated, and
**in that** the computing means (27) determine the specific isoenergy layers according to the at least one criterion, which is selected from the following set:

• the device (10) only irradiates the specific isoenergy layers where the particle beam for the irradiation of said layers has an energy lying above a minimum peak energy,
• the device (10) only irradiates the specific isoenergy layers where the particle beam for the irradiation of said layers has an energy lying below a maximum peak energy,
• the device (10) only irradiates the specific isoenergy layers which, according to the irradiation plan according to the first calculation pass, have the largest number of raster points of the isoenergy layers (7-9),
• the device (10) only irradiates the specific isoenergy layers which, according to the irradiation plan according to the first calculation pass, has the largest overall number of particles of the isoenergy layers (7-9),
• the device (10) only irradiates the specific isoenergy layers which, according to the irradiation plan according to the first calculation pass, makes the largest dose contribution to the overall dose to be applied in the target volume,
• the device (10) only irradiates the specific isoenergy layers which, according to the irradiation plan according to the first calculation pass, makes the largest contribution to a target function, which is calculated for determining the irradiation plan,
• the device (10) only irradiates the specific isoenergy layers which, according to the irradiation plan according to the first calculation pass, have the largest dose compensation error, wherein the dose compensation error specifies an error which occurs as a result of the non-irradiation of the specific isoenergy layers despite corresponding compensation by the irradiated isoenergy layers,
• the device (10) only irradiates the specific isoenergy layers which are determined proceeding from a set which, in accordance with the irradiation plan of the first pass, are to be irradiated,
wherein respectively a first target function value is generated on the basis of an irradiation plan which is generated under the assumption that only the set of the isoenergy layers is irradiated,
wherein in each case that isoenergy layer is added to the set which, in relation to the first target function value, modifies a second target function value to the greatest extent, which second target function value is determined on the basis of an irradiation plan generated under the assumption that only that isoenergy layer is irradiated in addition to the set,
wherein a further isoenergy layer is added to the set for as long as the change of the second target function value in relation to the first target function value is greater than a predetermined distance threshold,
• the device (10) only irradiates the specific isoenergy layers which are determined in accordance with any combination of the described criteria.

8. Device according to Claim 7,
**characterized**
**in that** the device (10) is embodied for performing the method according to one of Claims 1-6.

9. Particle irradiation unit comprising a device (10) according to Claim 7 or 8.

10. Computer program product, which comprises a program and is directly loadable into a memory of a programmable control apparatus of a particle irradiation unit (20), comprising program means for executing all steps of the method according to one of Claims 1-6 when the program is executed in the control apparatus of the particle irradiation unit (20).

11. Electronically readable data medium with electronically readable control information stored thereon which is configured in such a way that, when the data medium (29) is used in a control apparatus of a particle irradiation unit (20), it performs the method according to one of Claims 1-6.

**Revendications**

1. Procédé mis en œuvre par ordinateur de détermination d'un plan d'exposition d'une installation (20) d'exposition à des particules,
dans lequel on peut, par l'installation (20) d'exposition à des particules, exposer à un faisceau (16) de particules, en fonction du plan d'exposition, un volume (6) cible au sein d'un objet (14 ; 18) à examiner, dans lequel on prescrit le volume (6) cible et une répartition de dose définie à l'avance,
dans lequel on détermine le plan d'exposition dans une première passe de calcul, de manière à pouvoir appliquer le faisceau (16) de particules suivant la répartition de dose déterminée à l'avance au volume (6) cible, qui comprend plusieurs couches (7 à 9) d'iso-énergie,
**caractérisé**
**en ce que** l'on détermine le plan d'exposition dans une deuxième passe de calcul supplémentairement avec la condition que seules certaines couches d'iso-énergie sont exposées, et
**en ce que** l'on détermine certaines couches d'iso-énergie suivant au moins l'un des critères suivants :

• on n'expose que les certaines couches d'iso-énergie pour l'exposition desquelles le faisceau de particules a une énergie, qui est au-dessus d'une énergie limite minimum,
• on n'expose que les certaines couches d'iso-énergie pour l'exposition desquelles le faisceau de particules a une énergie, qui est en dessous d'une énergie limite maximum,
• on n'expose que les certaines couches d'iso-énergie, qui ont, suivant le plan d'exposition, après la première passe de calcul, le nombre le plus grand de points de trame des couches (7 à 9) d'iso-énergie,
• on n'expose que les certaines couches d'iso-énergie, qui ont, suivant le plan d'exposition, après la première passe de calcul, le nombre total de particules le plus grand des couches (7 à 9) d'iso-énergie,
• on n'expose que les certaines couches d'iso-énergie, qui ont, suivant le plan d'exposition, après la première passe de calcul, ont la contribution de dose la plus grande à la dose totale à appliquer au volume cible,
• on n'expose que les certaines couches d'iso-énergie, qui ont, suivant le plan d'exposition, après la première passe de calcul, la contribution la plus grande à une fonction cible, qui est calculée pour la détermination du plan d'exposition,
• on n'expose que les certaines couches d'iso-énergie, qui ont, suivant le plan d'exposition, après la première passe de calcul, le défaut de compensation de dose le plus grand, le défaut de compensation de dose indiquant un défaut, qui se produit par la non exposition des certaines couches d'iso-énergie, en dépit d'une compensation correspondante par les couches d'iso-énergie exposées,
• on détermine les certaines couches d'iso-énergie à partir d'un ensemble, qui doit être exposé suivant le plan d'exposition de la première passe,
dans lequel on établit, respectivement, une première valeur de fonction cible à l'aide d'un plan d'exposition, qui est établi avec l'hypothèse que seul l'ensemble des couches d'iso-énergie est exposé,
dans lequel on ajoute à l'ensemble la couche d'iso-énergie, qui, par rapport à la première valeur de fonction cible, modifie le plus une deuxième valeur de fonction cible, laquelle est déterminée à l'aide d'un plan d'exposition établi avec l'hypothèse que seule la couche d'iso-énergie est exposée supplémentairement à l'ensemble,
dans lequel on ajoute une autre couche d'iso-énergie à l'ensemble, tant que la variation de la deuxième valeur de fonction cible, par rapport à la première valeur de fonction cible, est plus grande qu'une valeur de seuil de distance donnée à l'avance,
• on n'expose que les certaines couches d'iso-énergie, qui sont déterminées suivant une combinaison quelconque des critères décrits.

2. Procédé suivant la revendication 1,
**caractérisé**
**en ce que** l'on forme un rapport des nombres ou de la proportion, conformément aux critères respectifs des certaines couches d'iso-énergie, à une somme des nombres ou des proportions correspondant aux critères respectifs de toutes les couches d'iso-énergie,

**en ce que** l'on choisit les certaines couches d'iso-énergie de manière à ce que le rapport soit au-dessus d'une valeur de seuil déterminée à l'avance, et

**en ce qu'**un retrait de l'une des certaines couches d'iso-énergie fait que le rapport passe en dessous de la valeur de seuil déterminée à l'avance.

3. Procédé suivant la revendication 1,
   **caractérisé**

   **en ce que** l'on forme une somme des nombres ou des proportions, conformément aux critères respectifs des certaines couches d'iso-énergie,

   **en ce que** l'on choisit les certaines couches d'iso-énergie de manière à ce que la somme soit au-dessus d'une valeur maximum déterminée à l'avance, et

   **en ce qu'**un retrait de l'une des certaines couches d'iso-énergie fait que la somme est en dessous de la valeur maximum déterminée à l'avance.

4. Procédé suivant l'une des revendications précédentes,
   **caractérisé**

   **en ce que** l'on prescrit une condition à observer par le plan d'exposition, et

   on ajoute au moins une ouche d'iso-énergie, suivant l'un des critères, aux certaines couches d'iso-énergie jusqu'à ce que le plan d'exposition satisfasse la condition.

5. Procédé suivant l'une des revendications précédentes,
   **caractérisé**

   **en ce que** le au moins un critère dépend d'une plage angulaire que forment deux champs, par lesquels le volume (6) cible est exposé et/ou en ce que le au moins un critère dépend de la géométrie du volume (6) cible.

6. Procédé suivant l'une des revendications précédentes,
   **caractérisé**

   **en ce que** la fonction cible comprend un terme de punition, en ce que le terme de punition est d'autant plus grand que plus de couches (7 à 9) d'iso-énergie sont exposées.

7. Dispositif de détermination d'un plan d'exposition d'une installation (20) d'exposition à des particules,

   dans lequel, par l'installation (20) d'exposition à des particules, un volume (6) cible au sein d'un objet (14 ; 18) à examiner est, en fonction du plan d'exposition, exposé à un faisceau (16) de particules,

   dans lequel le dispositif (10) comprend des moyens (26) d'entrée, des moyens (27) de calcul et des moyens (28) de sortie,

   dans lequel le volume (6) cible et une répartition de dose déterminée à l'avance peuvent être prescrits au dispositif (10) à l'aide des moyens (26) d'entrée,

   dans lequel les moyens (27) de calcul déterminent le plan d'exposition dans une première passe de calcul pour appliquer le faisceau (16) de particules suivant la répartition de dose déterminée à l'avance au volume (6) cible, qui comprend plusieurs couches (7 à 9) d'iso-énergie, et

   dans lequel les moyens (28) de sortie donnent le plan d'exposition,

   **caractérisé**

   **en ce qu'**il peut être prescrit au dispositif (10), à l'aide des moyens (26) d'entrée, au moins un critère pour les couches (7 à 9) d'iso-énergie,

   **en ce que** les moyens (27) de calcul déterminent le plan d'exposition dans une deuxième passe de calcul, supplémentairement avec la condition que le dispositif (10) ne peut soumettre à exposition que certaines des couches d'iso-énergie et en ce que les moyens (27) de calcul déterminent les certaines couches d'iso-énergie suivant le au moins un critère choisi dans l'ensemble suivant :

   • le dispositif (10) ne soumet à exposition que les certaines couches d'iso-énergie pour l'exposition desquelles le faisceau de particules a une énergie, qui est au-dessus d'une énergie limite minimum,
   • le dispositif (10) ne soumet que les certaines couches d'iso-énergie pour l'exposition desquelles le faisceau de particules a une énergie, qui est au-dessus d'une énergie limite minimum,
   • le dispositif (10) ne soumet que les certaines couches d'iso-énergie pour l'exposition desquelles le faisceau de particules a une énergie, qui est en dessous d'une énergie limite maximum,
   • le dispositif (10) ne soumet que les certaines couches d'iso-énergie, qui ont, suivant le plan d'exposition, après la première passe de calcul, le nombre le plus grand de points de trame des couches (7 à 9) d'iso-énergie,

• le dispositif (10) ne soumet que les certaines couches d'iso-énergie, qui ont, suivant le plan d'exposition, après la première passe de calcul, le nombre total de particules le plus grand des couches (7 à 9) d'iso-énergie,

• le dispositif (10) ne soumet que les certaines couches d'iso-énergie, qui ont, suivant le plan d'exposition, après la première passe de calcul, la contribution de dose la plus grande à la dose totale à appliquer au volume cible,

• le dispositif (10) ne soumet que les certaines couches d'iso-énergie, qui ont, suivant le plan d'exposition, après la première passe de calcul, la contribution la plus grande à une fonction cible, qui est calculée pour la détermination du plan d'exposition,

• le dispositif (10) ne soumet que les certaines couches d'iso-énergie, qui ont, suivant le plan d'exposition, après la première passe de calcul, le défaut de compensation de dose le plus grand, le défaut de compensation de dose indiquant un défaut, qui se produit par la non exposition des certaines couches d'iso-énergie, en dépit d'une compensation correspondante par les couches d'iso-énergie exposées,

• le dispositif (10) ne soumet que les certaines couches d'iso-énergie à partir d'un ensemble, qui doit être exposé suivant le plan d'exposition de la première passe,

dans lequel on établit, respectivement, une première valeur de fonction cible à l'aide d'un plan d'exposition, qui est établi avec l'hypothèse que seul l'ensemble des couches d'iso-énergie est exposé,

dans lequel on ajoute à l'ensemble la couche d'iso-énergie, qui, par rapport à la première valeur de fonction cible, modifie le plus une deuxième valeur de fonction cible, laquelle est déterminée à l'aide d'un plan d'exposition établi avec l'hypothèse que seule la couche d'iso-énergie, supplémentairement à l'ensemble, est exposée,

dans lequel on ajoute une autre couche d'iso-énergie à l'ensemble, tant que la variation de la deuxième valeur de fonction cible, par rapport à la première valeur de fonction cible, est plus grande qu'une valeur de seuil de distance donnée à l'avance,

• le dispositif (10) ne soumet que les certaines couches d'iso-énergie, qui sont déterminées suivant une combinaison quelconque des critères décrits.

8. Dispositif suivant la revendication 7,
**caractérisé**
**en ce que** le dispositif (10) est conformé pour effectuer le procédé suivant l'une des revendications 1 à 6.

9. Installation d'exposition à des particules, comprenant un dispositif (10) suivant la revendication 7 ou 8.

10. Produit de programme d'ordinateur, qui comprend un programme et qui peut être chargé directement dans une mémoire d'un dispositif de commande programmable d'une installation (20) d'exposition à des particules, comprenant des moyens de programme pour effectuer tous les stades du procédé suivant l'une des revendications 1 à 6, lorsque le programme est exécuté dans le dispositif de commande de l'installation (20) d'exposition à des particules.

11. Support de données déchiffrables électroniquement, comprenant des informations de commande déchiffrables électroniquement, qui y sont mises en mémoire et qui sont conformées de manière à effectuer le procédé suivant l'une des revendications 1 à 6, lors de l'utilisation du support (29) de données dans un dispositif de mémoire d'une installation (20) d'exposition à des particules.

FIG 1

EP 2 830 709 B1

FIG 2

# FIG 3

Start

S1 — Vorgeben eines Zielvolumens, einer Soll-Dosisverteilung, einer einzuhaltenden Planqualität und eines Kriteriums.

S2 — Bestimmen einer Menge von Isoenergieschichten abhängig von dem Zielvolumen.

S3 — Bestimmen eines Bestrahlungsplans anhand der Menge der Isoenergieschichten.

S4 — Erfüllt der Bestrahlungsplan die Planqualität?    nein

ja

S5 — Entfernen abhängig von dem Kriterium eine Isoenergieschicht aus der Menge.

Ende

# FIG 4

```
                    ┌──────────┐
                    │  Start   │
                    └──────────┘
                          │
                          ▼
┌─────────────────────────────────────────────────┐
│ Vorgeben eines Zielvolumens, einer Soll-Dosisverteilung, │── S1
│  einer einzuhaltenden Planqualität und eines Kriteriums. │
└─────────────────────────────────────────────────┘
                          │
                          ▼
┌─────────────────────────────────────────────────┐
│   Bestimmen von Isoenergieschichten abhängig von  │── S2
│              dem Zielvolumen.                      │
└─────────────────────────────────────────────────┘
                          │
                          ▼
┌─────────────────────────────────────────────────┐
│    Bestimmen eines Bestrahlungsplans anhand        │── S3
│          der Isoenergieschichten.                  │
└─────────────────────────────────────────────────┘
                          │
                          ▼
┌─────────────────────────────────────────────────┐
│   Bestimmen einer Menge von Isoenergieschichten.   │── S14
└─────────────────────────────────────────────────┘
                          │
                          ▼
┌─────────────────────────────────────────────────┐
│    Bestimmen eines Bestrahlungsplans anhand        │── S15
│       der Menge der Isoenergieschichten.           │
└─────────────────────────────────────────────────┘
                          │
                          ▼
┌─────────────────────────────────────────────────┐       nein
│   Erfüllt der Bestrahlungsplan die Planqualität?   │──────────┐── S16
└─────────────────────────────────────────────────┘          │
                          │ ja                                 │
                          ▼                                    │
┌─────────────────────────────────────────────────┐           │
│        Füge abhängig von dem Kriterium             │── S17     │
│    eine Isoenergieschicht zu der Menge hinzu.      │           │
└─────────────────────────────────────────────────┘           │
                                                               ▼
                                                        ┌──────────┐
                                                        │   Ende   │
                                                        └──────────┘
```